# EUROPEAN PATENT SPECIFICATION

(11) **EP 1 599 164 B1**
(45) Date of publication and mention of the grant of the patent: **08.04.2015**
(21) Application number: 04707401.8
(22) Date of filing: 02.02.2004
(51) Int. Cl.: A61K 39/02, G01N 33/569, C07K 16/12

(54) **METHODS FOR TREATING, PREVENTING AND DETECTING HELICOBACTER INFECTION**
VERFAHREN ZUR BEHANDLUNG, VORBEUGUNG UND NACHWEIS VON HELICOBACTER-INFEKTIONEN
PROCEDES DE TRAITEMENT, DE PREVENTION ET DE DIAGNOSTIC D'INFECTIONS A HELICOBACTER

(30) Priority: 03.02.2003 US 444190 P; 07.11.2003 US 518156 P
(43) Date of publication of application: 30.11.2005
(73) Proprietor: Cerebus Biologicals, Inc., Laramie, WY 82070 (US)
(72) Inventor: ELLIS, John, Saskatoon, Saskatchewan S7N 0M3 (CA); KRAKOWKA, George, Colombus, OH 434202 (US); EATON, Kathyrn, Saline, MI 48176 (US); FLORES, Joel, Newport News, VA 23608 (US)
(74) Representative: Mallalieu, Catherine Louise
(86) International application number: PCT/US2004/002867
(87) International publication number: WO 2004/069184

(56) References cited:
- US-A- 6 130 059
- US-A1- 2002 107 368
- US-A1- 2002 187 161
- DE GROOTE D ET AL: "Detection of "Candidatus Helicobacter suis" in gastric samples of pigs by PCR: comparison with other invasive diagnostic techniques." JOURNAL OF CLINICAL MICROBIOLOGY MAR 2000, vol. 38, no. 3, March 2000 (2000-03), pages 1131-1135, XP002408281 ISSN: 0095-1137
- KRAKOWKA STEVEN ET AL: "Isolation and preliminary characterization of a novel Helicobacter species from swine", AMERICAN JOURNAL OF VETERINARY RESEARCH, vol. 66, no. 6, June 2005 (2005-06), pages 938-944, ISSN: 0002-9645
- KRAKOWKA STEVEN ET AL: "Experimental induction of bacterial gastritis and gastric ulcer disease in gnotobiotic swine inoculated with porcine Helicobacter-like species", AMERICAN JOURNAL OF VETERINARY RESEARCH, vol. 66, no. 6, June 2005 (2005-06), pages 945-952, ISSN: 0002-9645
- KRAKOWKA S ET AL: "Reproduction of severe gastroesophageal ulcers (GEU) in gnotobiotic swine infected with porcine Helicobacter pylori-like bacteria.", VETERINARY PATHOLOGY NOV 2006, vol. 43, no. 6, November 2006 (2006-11), pages 956-962, ISSN: 0300-9858
- DIETERICH C ET AL: "Urease-based mucosal immunization against Helicobacter heilmannii infection induces corpus atrophy in mice.", INFECTION AND IMMUNITY NOV 1999, vol. 67, no. 11, November 1999 (1999-11), pages 6206-6209, ISSN: 0019-9567
- LEE A: "Animal models and vaccine development.", BAILLIÈRE'S CLINICAL GASTROENTEROLOGY SEP 1995, vol. 9, no. 3, September 1995 (1995-09), pages 615-632, ISSN: 0950-3528
- FRIENDSHIP R ET AL: "Helicobacter infection: What should a swine practitioner know", SWINE HEALTH AND PRODUCTION, DES MOINES, IA, US, vol. 7, no. 4, 1 January 1999 (1999-01-01) , pages 167-172, XP007919112, ISSN: 1066-4963

## Description

### TECHNICAL FIELD

The present invention relates generally to bacterial immunogens. In particular, the invention pertains to *Helicobacter cerdo,* a new pathogen isolated from swine, and methods of treating, preventing and diagnosing *Helicabacter* infection using immunogenic proteins and nucleic acids derived from *H. cerdo.*

### BACKGROUND

Gastric disease is an important cause of morbidity and economic loss in swine-rearing operations (O'Brien, J. (1992) "Gastric ulcers" p.680. In A. D. Leman, B. E. Straw, W. L. Mengeling, and S. D. D'Allaire (ed), Diseases of swine. Wolfe, London, United Kingdom). Although the cause of porcine gastric disease has not been previously established, it is most often attributed to diet and/or stress (O'Brien, J. (1992) "Gastric ulcers" p.680. In A. D. Leman, B. E. Straw, W. L. Mengeling, and S. D. D'Allaire (ed), Diseases of swine. Wolfe, London, United Kingdom).

In 1984, *Helicobacter pylori* (Hp) emerged as an etiologic agent in human gastritis/ulcer disease following the documentation of this agent in patients with gastritis (Marshall and Warren (1984) Lancet 1:1311-1314). Hp is now universally recognized as one of the primary gastric pathogens and the study of this bacterial species and the spectrum of diseases associated with it has become a major focus in human gastroenterology (Suerbaum and Michetti (2002) N. Eng. J. Med. 347:1175-1186). Hp is causally associated with chronic superficial (active) type B gastritis (Buck (1990) Clin. Micro. Rev. 3:1-12; Blaser (1992) Gasteroenterol. 102:720-727; Consensus Statement, 1994, NSAID), independent gastric ulceration (Peterson (1991) N. Eng. J. Med. 324:1043-1047; Moss and Calam (1992) Gut 33:289- 292; Leung et al. (1992) Am. J. Clin. Pathol. 98:569 574; Forbes et al. (1994) Lancet 343:258-260), atrophic gastritis (Nomura et al. (1991) N. Engl. J. Med. 325:1132-1136; Parsonnet et al. (1991) JNCI 83:640-643; Sipponen (1992) Drugs 52:799-804, 1996), and gastric MALT lymphoma (Rodriguez et al. (1993) Acta Gastro-Enterol. Belg. 56 (suppl):47; Eidt et al. (1994) J. Clin. Pathos. 47:436-439).

Multiple agent antimicrobial therapies have been available for human Hp for more than a decade. These therapies can be expensive, cumbersome to administer, and often do not completely cure the disease. Such therapies would be impractical in domestic livestock. Moreover, injudicious use of antimicrobials promotes emergence of antibiotic-resistant strains of Hp and Hp resistance to metronidazole and clarirythromycin has increased (Michetti, (1997) Gut 41:728-730). Additionally, the use of antibiotics in food animals is undesirable.

Attempts to treat Hp infection in humans using immunotherapy rather than chemotherapy has been largely unsuccessful. In particular, induction of immunity which mimics the "natural" immune response of convalescent infected humans has not been successful since human Hp infection can persist indefinitely in spite of a strong immune response to Hp (Lee (1996) Gastroenterol. 110:2003-2006). In mice, protection has been achieved with sonicates or recombinant proteins such as ureA and ureB, vacA and GroEL, given orally with cholera toxin (CT) and heat labile toxin (LT) as adjuvants. The focus has been primarily upon the use of purified and/or recombinant bacterial proteins as target immunogens in vaccine development programs. In general, inconsistent and only partial protection has been achieved. In rodent systems, mucosal vaccination assisted by CT or LT has emerged as the favored route, notwithstanding the fact that these species are highly resistant to toxic effects of CT/LT and the resultant rodent data does not directly translate into the human or swine experience.

In particular, in piglets immunized and then challenged with Hp, the strongest pre-challenge indicator of efficacy is the level and presence of Hp-specific serum/salivary IgG, not IgA (Eaton and Krakowka (1992) Gastroenterol. 103:1580-1586). Parenteral vaccination stimulates a strong IgG response; oral vaccination does not. Parenteral immunization was completely protective in 50% of the piglets immunized subcutaneously and in 60% of piglets immunized intraperitoneally (Eaton et al. (1998) J. Inflect. Dis. 178:1399-1405). In contrast, oral vaccination with: 1) live bacteria (cleared with antimicrobials prior to challenge), 2) whole intact killed bacteria, 3) whole bacterial sonicates and 4) whole bacterial sonicates with mucosal LT adjuvant failed to provide a single instance (0 of 27 piglets or 0%) of protection. Bacterial cfu were reduced compared to controls but the levels of reduction did not reach statistical significance. Thus, in the porcine model of Hp colonization and acute gastritis, the parenteral route of vaccination appears to be superior to the oral route in both absolute (infected versus uninfected after challenge) and relative (bacterial cfu in vaccinates versus nonvaccinated controls) measures of antimicrobial efficacy.

Dieterich et al (Infection and Immunity, 1999, 67: 6206-6209) disclose that immunisation of mice with *Helicobacter heilmannii* urease B or *Helicobacter pylori* urease protects against *H. heilmannii* infection and significantly reduces a preexisting infection.

### SUMMARY OF THE INVENTION

The present invention is based on the discovery of a novel *Helicobacter* pathogen isolated from swine exhibiting gastritis/ulcer disease. This organism has been named *Helicobacter cerdo* (He) by the inventors herein. This organism has been shown by the inventors to cause gastric disease in young piglets that is similar to Hp-associated active gastritis in humans.

Subunit vaccines, including antigens and mixtures of antigens derived from *H*. *cerdo,* provide protection against subsequent infection with *Helicobacter species,* such as *H. pylori* and *H. cerdo.* The present invention provides a safe, efficacious and economical method of treating and/or preventing He infection in swine.

Accordingly, in one embodiment, the subject invention is directed to a composition comprising a pharmaceutically acceptable vehicle and at least one *Helicobacter cerdo* immunogen. In certain embodiments, the at least one *H. cerdo* immunogen is provided in an *H. cerdo* lysate, such as a lysate produced by proteolytic digestion of *H. cerdo* bacteria. In additional embodiments, the composition further comprises an adjuvant.

In another embodiment, the invention is directed to methods of treating or preventing a *Helicobacter* infection in a vertebrate subject comprising administering to the subject a therapeutically effective amount of a composition as described above. In certain embodiments, the vertebrate subject is a porcine subject In additional embodiments, the *Helicobacter* infection is a *Helicobacter cerdo* infection. In yet further embodiments, the composition is administered parenterally.

In yet another embodiment, the invention is directed to methods of treating or preventing a *Helicobacter cerdo* infection in a porcine subject comprising parenterally administering to the subject a therapeutically effective amount of a composition as described above.

In another embodiment, the invention is directed to a method of producing a composition comprising:
(a) providing at least one *Helicobacter cerdo* immunogen; and
(b) combining the *H. cerdo* immunogen with a pharmaceutically acceptable vehicle.

In certain embodiments, the at least one *H. cerdo* immunogen is provided in an *H. cerdo* lysate, such as an *H. cerdo* lysate produced by proteolytic digestion *of H. cerdo* bacteria. In additional embodiments, an adjuvant is also provided.

In yet another embodiment, the invention is directed to a method of detecting *Helicobacter* infection in a subject comprising:
(a) providing a biological sample from the subject; and
(b) reacting the biological sample with at least one *H. cerdo* immunogen, under conditions which allow *Helicobacter* antibodies, when present in the biological sample, to bind with the immunogen(s),
   thereby detecting the presence or absence of *Helicobacter* infection in the subject.
   In certain embodiments, the method further comprises:
(c) removing unbound antibodies;
(d) providing one or more moieties capable of associating with the bound antibodies; and
(e) detecting the presence or absence of the one or more moieties, thereby detecting the presence or absence of *H. cerdo* infection.

In certain embodiments, the detectable label is a fluorescer or an enzyme. In additional embodiments, the at least one immunogen is provided in an *H. cerdo* lysate. In still further embodiments, the biological sample is a porcine serum sample.

In additional embodiments, the invention is directed to a method of detecting *Helicobacter cerdo* infection in a porcine subject comprising:
(a) providing a biological sample from the subject; and
(b) reacting the biological sample with at least one *H. cerdo* immunogen, under conditions which allow *H. cerdo* antibodies, when present in the biological sample, to bind with the immunogen(s),
(c) removing unbound antibodies;
(d) providing one or more moieties capable of associating with the bound antibodies; and
(e) detecting the presence or absence of the one or more moieties, thereby detecting the presence or absence of *H. cerdo* infection.

In still further embodiments, the invention is directed to an antibody specific for a *Helicobacter cerdo* immunogen. In certain embodiments, the antibody is a polyclonal antibody. In other embodiments, the antibody is a monoclonal antibody.

In another embodiment, the invention is directed to a *Helicobacter cerdo* lysate comprising at least one *H. cerdo* immunogen. In certain embodiments, the *H. cerdo* lysate is produced by proteolytic digestion of *H. cerdo* bacteria.

These and other embodiments of the subject invention will readily occur to those of skill in the art in view of the disclosure herein.

### BRIEF DESCRIPTION OF THE DRAWINGS

Figure 1 shows SDS-PAGE profiles of intact and digested *H. pylori* and *H. cerdo* preparations. The ">" in the figure illustrates bands present in *H. pylori* and absent from *H. cerdo.* The "]" indicates low molecular weight protease digest products.
Figures 2A and 2B show SDS-PAGE separations of intact *H. cerdo* (2A) and an *H. cerdo* digest (2B). An increased amount of low molecular weight material (<) is seen in the digested preparation.
Figures 3A and 3B show a Western blot analysis of intact *H. cerdo* (3A) and an *H*. *cerdo* digest (3B) separated on a native, non-reducing gel.
Figures 4A and 4B show a Western blot analysis of the antibody reactivity profile against intact *H. cerdo* (4A) and an *H. cerdo* digest (4B). An increased amount of low molecular weight material is seen in the digest (indicated by ]). Increased staining intensity is also seen (■), as well as additional immunoreactive bands (<).

### DETAILED DESCRIPTION OF THE INVENTION

The practice of the present invention will employ, unless otherwise indicated, conventional techniques of molecular biology, microbiology, bacteriology, recombinant DNA technology, and immunology, which are within the skill of the art. Such techniques are explained fully in the literature. See, e.g., Sambrook, Fritsch & Maniatis, Molecular Cloning: A Laboratory Manual, Second Edition (1989); DNA Cloning, Vols. I and II (D.N. Glover ed. 1985); Oligonucleotide Synthesis (M.J. Gait ed. 1984); Nucleic Acid Hybridization (B.D. Hames & S.J. Higgins eds. 1984); Animal Cell Culture (R.K. Freshney ed. 1986); Immobilized Cells and Enzymes (IRL press, 1986); Perbal, B., A Practical Guide to Molecular Cloning (1984); the series, Methods In Enzymology (S. Colowick and N. Kaplan eds., Academic Press, Inc.); and Handbook of Experimental Immunology, Vols. I-IV (D.M. Weir and C.C. Blackwell eds., 1986, Blackwell Scientific Publications).

All publications, patents and patent applications cited herein, whether *supra* or *infra,* are hereby incorporated by reference in their entirety.

### 1. DEFINITIONS

In describing the present invention, the following terms will be employed, and are intended to be defined as indicated below.

It must be noted that, as used in this specification and the appended claims, the singular forms "a", "an" and "the" include plural referents unless the content clearly dictates otherwise. Thus, for example, reference to "an *H. cerdo* immunogen" includes a mixture of two or more such immunogens, and the like.

By *"Helicobacter* infection" is meant any disorder caused by a *Helicobacter* bacterium, including without limitation, *H. cerdo, H. pylori* and *H. heilmannii,* such as, but not limited to, chronic superficial (active) type B gastritis, independent gastric ulceration, peptic, gastric and duodenal ulcers, gastroesophageal ulceration (GEU), proventricular ulcers, ulcerative gastric hemorrhage, atrophic gastritis, and carcinoma including gastric MALT lymphoma. The term also intends subclinical disease, e.g., where *Helicobacter* infection is present but clinical symptoms of disease have not yet manifested themselves. Subjects with subclinical disease can be asymptomatic but are nonetheless at a considerable risk of developing peptic ulcers and/or gastric adenocarcinomas. For a review of *Helicobacter*-associated diseases, see, Telford et al., Trends in Biotech. (1994) 12:420-426 and Blaser, M.J., Scientific American (February 1996):104-107.

By "an *H. cerdo* lysate" is meant an extract or lysate derived from an *H. cerdo* whole bacterium which includes one or more *H. cerdo* immunogenic polypeptides, as defined below. The term therefore is intended to encompass crude extracts that contain several *H. cerdo* immunogens as well as relatively purified compositions derived from such crude lysates which include only one or few such immunogens. Such lysates are prepared using techniques well known in the art, described further below.

Representative immunogens that may be present in such lysates, either alone or in combination, include immunogens with one or more epitopes derived from *H*. *cerdo* adhesins such as, but not limited to, *H. cerdo* immunogens corresponding to a 20 kDa N-acetyl-neuraminillactose-binding fibrillar haemagglutinin (HpaA), a 63 kDa protein that binds phosphatidylethanolamine and gangliotetraosyl ceramide, and a conserved fimbrial pilus-like structure as found in *H. pylori.* See, e.g., Telford et al., Trends in Biotech. (1994) 12:420-426 for a description of these antigens.

Other immunogens that may be present in the lysate include immunogens with one or more epitopes derived from any of the various flagellins corresponding to the *H. pylori* flagellins known as the major flagellin, FlaA and the minor flagellin, FlaB. The flagella of *H. pylori* are composed of FlaA and FlaB, each with molecular weights of approximately 55 kDa. Immunogens from *H. cerdo* corresponding to either or both of FlaA and/or FlaB may be used in the lysates of the present invention.

Another representative *H. cerdo* immunogen is an immunogen corresponding to *H. pylori* urease which is associated with the outer membrane and the periplasmic space of the bacterium. The *H. pylori* holoenzyme is a large complex made up of two subunits of 26.5 kDa (UreA) and 61 kDa (UreB), respectively. *H. cerdo* immunogens with epitopes derived from the holoenzyme, either of the subunits, or a combination of the three, can be present in the compositions.

Another representative immunogen that may be present in the lysate or used in further purified form includes the *H. cerdo* protein corresponding to the *H. pylori* heat shock protein known as "hsp60." See, e.g., International Publication No. WO 93/18150.

Additionally, the *H. cerdo* cytotoxin corresponding to the *H. pylori* cytotoxin may also be present. This cytotoxin is an ion transport ATPase which includes 87 kDa (monomer) and 972 kDa (decamer) forms. One cytotoxin is commonly termed "CagA." CagA is associated with the immunodominant antigen and is expressed on the bacterial surface. The DNA and corresponding amino acid sequences for *H. pylori* CagA are known. See, e.g., International Publication No. WO 93/18150, published 16 September 1993. The native protein shows interstrain size variability due to the presence of a variable number of repeats of a 102 bp DNA segment that encodes repeats of a proline-rich amino acid sequence. See, Covacci et al., Proc. Natl. Acad. Sci. USA (1993) 90:5791-5795. Accordingly, the reported molecular weight of CagA ranges from about 120-135 kDa. Hence, if CagA is present in the lysate, it can be present as any of the various CagA variants, fragments thereof and muteins thereof, which retain activity.

Yet another immunogen that may be present in the lysate includes the *H*. *cerdo* VacA protein. The DNA and corresponding amino acid sequences for *H. pylori* VacA are known and reported in, e.g., International Publication No. WO 93/18150, published 16 September 1993. The gene for the VacA polypeptide encodes a precursor of about 140 kDa that is processed to an active molecule of about 90-100 kDa. This molecule, in turn, is slowly proteolytically cleaved to generate two fragments that copurify with the intact 90 kDa molecule. See, Telford et al., Trends in Biotech. (1994) 12:420-426. Thus, the lysate can include the precursor protein, as well as the processed active molecule, active proteolytic fragments thereof or portions or muteins thereof, which retain biological activity.

It is to be understood that the lysate can also include other immunogens not specifically described herein.

The term "polypeptide" when used with reference to an *H. cerdo* immunogen, such as VacA, CagA or any of the other immunogens described above, refers to a VacA, CagA etc., whether native, recombinant or synthetic, which is derived from any *H. cerdo* strain. The polypeptide need not include the full-length amino acid sequence of the reference molecule but can include only so much of the molecule as necessary in order for the polypeptide to retain immunogenicity and/or the ability to treat or prevent *H. cerdo* infection, as described below. Thus, only one or few epitopes of the reference molecule need be present. Furthermore, the polypeptide may comprise a fusion protein between the full-length reference molecule or a fragment of the reference molecule, and another protein that does not disrupt the reactivity of the *H. cerdo* polypeptide. It is readily apparent that the polypeptide may therefore comprise the full-length sequence, fragments, truncated and partial sequences, as well as analogs and precursor forms of the reference molecule. The term also intends deletions, additions and substitutions to the reference sequence, so long as the polypeptide retains immunogenicity.

Thus, the full-length proteins and fragments thereof, as well as proteins with modifications, such as deletions, additions and substitutions (either conservative or non-conservative in nature), to the native sequence, are intended for use herein, so long as the protein maintains the desired activity. These modifications may be deliberate, as through site-directed mutagenesis, or may be accidental, such as through mutations of hosts which produce the proteins or errors due to PCR amplification. Accordingly, active proteins substantially homologous to the parent sequence, e.g., proteins with 70...80...85...90...95...98...99% etc. identity that retain the biological activity, are contemplated for use herein.

The term "analog" refers to biologically active derivatives of the reference molecule, or fragments of such derivatives, that retain activity, as described above. In general, the term "analog" refers to compounds having a native polypeptide sequence and structure with one or more amino acid additions, substitutions and/or deletions, relative to the native molecule. Particularly preferred analogs include substitutions that are conservative in nature, i.e., those substitutions that take place within a family of amino acids that are related in their side chains. Specifically, amino acids are generally divided into four families: (1) acidic -- aspartate and glutamate; (2) basic -- lysine, arginine, histidine; (3) non-polar -- alanine, valine, leucine, isoleucine, proline, phenylalanine, methionine, tryptophan; and (4) uncharged polar -- glycine, asparagine, glutamine, cysteine, serine threonine, tyrosine. Phenylalanine, tryptophan, and tyrosine are sometimes classified as aromatic amino acids. For example, it is reasonably predictable that an isolated replacement of leucine with isoleucine or valine, an aspartate with a glutamate, a threonine with a serine, or a similar conservative replacement of an amino acid with a structurally related amino acid, will not have a major effect on the biological activity. For example, the polypeptide of interest may include up to about 5-10 conservative or non-conservative amino acid substitutions, or even up to about 15-25 or 50 conservative or non-conservative amino acid substitutions, or any number between 5-50, so long as the desired function of the molecule remains intact.

A "purified" protein or polypeptide.is a protein which is recombinantly or synthetically produced, or isolated from its natural host, such that the amount of protein present in a composition is substantially higher than that present in a crude preparation. In general, a purified protein will be at least about 50% homogeneous and more preferably at least about 80% to 90% homogeneous.

By "biologically active" is meant an *H. cerdo* protein that elicits an immunological response, as defined below.

By "epitope" is meant a site on an antigen to which specific B cells and T cells respond. The term is also used interchangeably with "antigenic determinant" or "antigenic determinant site." An epitope can comprise 3 or more amino acids in a spatial conformation unique to the epitope. Generally, an epitope consists of at least 5 such amino acids and, more usually, consists of at least 8-10 such amino acids. Methods of determining spatial conformation of amino acids are known in the art and include, for example, x-ray crystallography and 2-dimensional nuclear magnetic resonance. Furthermore, the identification of epitopes in a given protein is readily accomplished using techniques well known in the art, such as by the use of hydrophobicity studies and by site-directed serology. See, also, Geysen et al., Pro. Natl. Acad. Sci. USA (1984) 81:3998-4002 (general method of rapidly synthesizing peptides to determine the location of immunogenic epitopes in a given antigen); U.S. Patent No. 4,708,871 (procedures for identifying and chemically synthesizing epitopes of antigens); and Geysen et al., Molecular Immunology (1986) 23:709-715 (technique for identifying peptides with high affinity for a given antibody). Antibodies that recognize the same epitope can be identified in a simple immunoassay showing the ability of one antibody to block the binding of another antibody to a target antigen.

An "immunological response" to a composition or vaccine is the development in the host of a cellular and/ or antibody-mediated immune response to the composition or vaccine of interest. Usually, an "immunological response" includes but is not limited to one or more of the following effects: the production of antibodies, B cells, helper T cells, suppressor T cells, and/or cytotoxic T cells and/or γδ T cells, directed specifically to an antigen or antigens included in the composition or vaccine of interest. Preferably, the host will display a protective immunological response to the *H. cerdo* immunogen(s) in question, e.g., the host will be protected from subsequent infection by the pathogen and such protection will be demonstrated by either a reduction or lack of symptoms normally displayed by an infected host or a quicker recovery time.

The terms "immunogenic" protein or polypeptide refer to an amino acid sequence which elicits an immunological response as described above. An "immunogenic" protein or polypeptide, as used herein, includes the full-length sequence of the particular *H. cerdo* immunogen in question, including any precursor and mature forms, analogs thereof, or immunogenic fragments thereof. By "immunogenic fragment" is meant a fragment of the *H. cerdo* immunogen in question which includes one or more epitopes and thus elicits the immunological response described above.

Immunogenic fragments, for purposes of the present invention, will usually be at least about 2 amino acids in length, more preferably about 5 amino acids in length, and most preferably at least about 10 to 15 amino acids in length. There is no critical upper limit to the length of the fragment, which could comprise nearly the full-length of the protein sequence, or even a fusion protein comprising two or more epitopes of the *H. cerdo* immunogen in question.

"Homology" refers to the percent identity between two polynucleotide or two polypeptide moieties. Two DNA, or two polypeptide sequences are "substantially homologous" to each other when the sequences exhibit at least about 50% , preferably at least about 75%, more preferably at least about 80%-85%, preferably at least about 90%, and most preferably at least about 95%-98% sequence identity over a defined length of the molecules. As used herein, substantially homologous also refers to sequences showing complete identity to the specified DNA or polypeptide sequence.

In general, "identity" refers to an exact nucleotide-to-nucleotide or amino acid-to-amino acid correspondence of two polynucleotides or polypeptide sequences, respectively. Percent identity can be determined by a direct comparison of the sequence information between two molecules by aligning the sequences, counting the exact number of matches between the two aligned sequences, dividing by the length of the shorter sequence, and multiplying the result by 100. Readily available computer programs can be used to aid in the analysis, such as ALIGN, Dayhoff, M.O. in Atlas of Protein Sequence and Structure M.O. Dayhoff ed., 5 Suppl. 3:353-358, National Biomedical Research Foundation, Washington, DC, which adapts the local homology algorithm of Smith and Waterman *Advances in Appl. Math.* 2:482-489, 1981 for peptide analysis. Programs for determining nucleotide sequence identity are available in the Wisconsin Sequence Analysis Package, Version 8 (available from Genetics Computer Group, Madison, WI) for example, the BESTFIT, FASTA and GAP programs, which also rely on the Smith and Waterman algorithm. These programs are readily utilized with the default parameters recommended by the manufacturer and described in the Wisconsin Sequence Analysis Package referred to above. For example, percent identity of a particular nucleotide sequence to a reference sequence can be determined using the homology algorithm of Smith and Waterman with a default scoring table and a gap penalty of six nucleotide positions.

Another method of establishing percent identity in the context of the present invention is to use the MPSRCH package of programs copyrighted by the University of Edinburgh, developed by John F. Collins and Shane S. Sturrok, and distributed by IntelliGenetics, Inc. (Mountain View, CA). From this suite of packages the Smith-Waterman algorithm can be employed where default parameters are used for the scoring table (for example, gap open penalty of 12, gap extension penalty of one, and a gap of six). From the data generated the "Match" value reflects "sequence identity." Other suitable programs for calculating the percent identity or similarity between sequences are generally known in the art, for example, another alignment program is BLAST, used with default parameters. For example, BLASTN and BLASTP can be used using the following default parameters: genetic code = standard; filter = none; strand = both; cutoff = 60; expect = 10; Matrix = BLOSUM62; Descriptions = 50 sequences; sort by = HIGH SCORE; Databases = non-redundant, GenBank + EMBL + DDBJ + PDB + GenBank CDS translations + Swiss protein + Spupdate + PIR. Details of these programs are well known in the art.

Alternatively, homology can be determined by hybridization of polynucleotides under conditions which form stable duplexes between homologous regions, followed by digestion with single-stranded-specific nuclease(s), and size determination of the digested fragments. DNA sequences that are substantially homologous can be identified in a Southern hybridization experiment under, for example, stringent conditions, as defined for that particular system. Defining appropriate hybridization conditions is within the skill of the art. See, e.g., Sambrook et al., *supra; DNA Cloning, supra; Nucleic Acid Hybridization, supra.*

A "coding sequence" or a sequence which "encodes" a selected polypeptide, is a nucleic acid molecule which is transcribed (in the case of DNA) and translated (in the case of mRNA) into a polypeptide *in vitro* or *in vivo* when placed under the control of appropriate regulatory sequences. The boundaries of the coding sequence are determined by a start codon at the 5' (amino) terminus and a translation stop codon at the 3' (carboxy) terminus. A transcription termination sequence may be located 3' to the coding sequence.

By "vector" is meant any genetic element, such as a plasmid, phage, transposon, cosmid, chromosome, virus, virion, etc., which is capable of replication when associated with the proper control elements and which can transfer gene sequences to cells. Thus, the term includes cloning and expression vehicles, as well as viral vectors.

By "recombinant vector" is meant a vector that includes a heterologous nucleic acid sequence which is capable of expression *in vitro* or *in vivo.*

The term "transfection" is used to refer to the uptake of foreign DNA by a cell, and a cell has been "transfected" when exogenous DNA has been introduced inside the cell membrane. A number of transfection techniques are generally known in the art. *See, e.g.,* Graham et al. (1973) Virology, 52 :456, Sambrook et al. (1989) Molecular Cloning, a laboratory manua/, Cold Spring Harbor Laboratories, New York, Davis et al. (1986) Basic Methods in Molecular Biology, Elsevier, and Chu et al. (1981) Gene 13:197. Such techniques can be used to introduce one or more exogenous DNA moieties into suitable host cells.

The term "heterologous" as it relates to nucleic acid sequences such as coding sequences and control sequences, denotes sequences that are not normally joined together, and/or are not normally associated with a particular cell. Thus, a "heterologous" region of a nucleic acid construct or a vector is a segment of nucleic acid within or attached to another nucleic acid molecule that is not found in association with the other molecule in nature. For example, a heterologous region of a nucleic acid construct could include a coding sequence flanked by sequences not found in association with the coding sequence in nature. Another example of a heterologous coding sequence is a construct where the coding sequence itself is not found in nature (e.g., synthetic sequences having codons different from the native gene). Similarly, a cell transformed with a construct which is not normally present in the cell would be considered heterologous for purposes of this invention. Allelic variation or naturally occurring mutational events do not give rise to heterologous DNA, as used herein.

A "nucleic acid" sequence refers to a DNA or RNA sequence. The term captures sequences that include any of the known base analogues of DNA and RNA such as, but not limited to 4-acetylcytosine, 8-hydroxy-N6-methyladenosine, aziridinylcytosine, pseudoisocytosine, 5-(carboxyhydroxyl-methyl) uracil, 5-fluorouracil, 5-bromouracil, 5-carboxymethylaminomethyl-2-thiouracil, 5-carboxymethyl-aminomethyluracil, dihydrouracil, inosine, N6-isopentenyladenine, 1-methyladenine, 1-methylpseudo-uracil, 1-methylguanine, 1-methylinosine, 2,2-dimethyl-guanine, 2-methyladenine, 2-methylguanine, 3-methyl-cytosine, 5-methylcytosine, N6-methyladenine, 7-methylguanine, 5-methylaminomethyluracil, 5-methoxy-amino-methyl-2-thiouracil, beta-D-mannosylqueosine, 5'-methoxycarbonylmethyluracil, 5-methoxyuracil, 2-methylthio-N6-isopentenyladenine, uracil-5-oxyacetic acid methylester, uracil-5-oxyacetic acid, oxybutoxosine, pseudouracil, queosine, 2-thiocytosine, 5-methyl-2-thiouracil, 2-thiouracil, 4-thiouracil, 5-methyluracil, -uracil-5-oxyacetic acid methylester, uracil-5-oxyacetic acid, pseudouracil, queosine, 2-thiocytosine, and 2,6-diaminopurine.

The term DNA "control sequences" refers collectively to promoter sequences, polyadenylation signals, transcription termination sequences, upstream regulatory domains, origins of replication, internal ribosome entry sites ("IRES"), enhancers, and the like, which collectively provide for the replication, transcription and translation of a coding sequence in a recipient cell. Not all of these control sequences need always be present so long as the selected coding sequence is capable of being replicated, transcribed and translated in an appropriate host cell.

The term "promoter" is used herein in its ordinary sense to refer to a nucleotide region comprising a DNA regulatory sequence, wherein the regulatory sequence is derived from a gene which is capable of binding RNA polymerase and initiating transcription of a downstream (3'-direction) coding sequence. Transcription promoters can include "inducible promoters" (where expression of a polynucleotide sequence operably linked to the promoter is induced by an analyte, cofactor, regulatory protein, etc.), "repressible promoters" (where expression of a polynucleotide sequence operably linked to the promoter is induced by an analyte, cofactor, regulatory protein, etc.), and "constitutive promoters".

"Operably linked" refers to an arrangement of elements wherein the components so described are configured so as to perform their usual function. Thus, control sequences operably linked to a coding sequence are capable of effecting the expression of the coding sequence. The control sequences need not be contiguous with the coding sequence, so long as they function to direct the expression thereof. Thus, for example, intervening untranslated yet transcribed sequences can be present between a promoter sequence and the coding sequence and the promoter sequence can still be considered "operably linked" to the coding sequence.

For the purpose of describing the relative position of nucleotide sequences in a particular nucleic acid molecule throughout the instant application, such as when a particular nucleotide sequence is described as being situated "upstream," "downstream," "3 prime (3')" or "5 prime (5')" relative to another sequence, it is to be understood that it is the position of the sequences in the "sense" or "coding" strand of a DNA molecule that is being referred to as is conventional in the art.

By "vertebrate subject" is meant any member of the subphylum chordata, including, without limitation, mammals such as cattle, sheep, pigs, goats, horses, and humans; domestic animals such as dogs and cats; and birds, including domestic, wild and game birds such as cocks and hens including chickens, turkeys and other gallinaceous birds; and fish. The term does not denote a particular age. Thus, both adult and newborn animals, as well as fetuses, are intended to be covered.

The terms "effective amount" or "therapeutically effective amount" of a composition or agent, as provided herein, refer to a nontoxic but sufficient amount of the composition or agent to provide the desired "therapeutic effect," such as to elicit an immune response as described above, preferably preventing, reducing or reversing symptoms associated with the *Helicobacter* infection. This effect can be to alter a component of a disease (or disorder) toward a desired outcome or endpoint, such that a subject's disease or disorder shows improvement, often reflected by the amelioration of a sign or symptom relating to the disease or disorder. For example, a representative therapeutic effect can render the subject negative for *Helicobacter* infection when gastric mucosa is cultured for the particular *Helicobacter* species in question, such as *H. cerdo.* Similarly, biopsies indicating lowered IgG, IgM and IgA antibody production directed against the *Helicobacter* species in question, such as *H. cerdo* are an indication of a therapeutic effect. Similarly, decreased serum antibodies against the *Helicobacter* species in question are indicative of a therapeutic effect. Reduced gastric inflammation is also indicative of a therapeutic effect. The exact amount required will vary from subject to subject, depending on the species, age, and general condition of the subject, the severity of the condition being treated, and the particular components of the composition administered, mode of administration, and the like. An appropriate "effective" amount in any individual case may be determined by one of ordinary skill in the art using routine experimentation.

"Treatment" or "treating" *Helicobacter* infection includes: (1) preventing the *Helicobacter* disease, or (2) causing disorders related to *Helicobacter* infection to develop or to occur at lower rates in a subject that may be exposed to *Helicobacter,* such as *H. cerdo,* (3) reducing the amount of *Helicobacter* present in a subject, and/or reducing the symptoms associated with *Helicobacter* infection.

As used herein, a "biological sample" refers to a sample of tissue or fluid isolated from an individual, including but not limited to, for example, blood, plasma, serum, fecal matter, urine, bone marrow, bile, spinal fluid, lymph fluid, samples of the skin, external secretions of the skin, respiratory, intestinal, and genitourinary tracts, samples derived from the gastric epithelium and gastric mucosa, tears, saliva, milk, blood cells, organs, biopsies and also samples *of in vitro* cell culture constituents including but not limited to conditioned media resulting from the growth of cells and tissues in culture medium, e.g., recombinant cells, and cell components.

As used herein, the terms "label" and "detectable label" refer to a molecule capable of detection, including, but not limited to, radioactive isotopes, fluorescers, chemiluminescers, enzymes, enzyme substrates, enzyme cofactors, enzyme inhibitors, chromophores, dyes, metal ions, metal sols, ligands (e.g., biotin or haptens) and the like. The term "fluorescer" refers to a substance or a portion thereof which is capable of exhibiting fluorescence in the detectable range. Particular examples of labels which may be used under the invention include fluorescein, rhodamine, dansyl, umbelliferone, Texas red, luminol, acradimum esters, NADPH and α-β-galactosidase.

### 2. MODES OF CARRYING OUT THE INVENTION

Before describing the present invention in detail, it is to be understood that this invention is not limited to particular formulations or process parameters as such may, of course, vary. It is also to be understood that the terminology used herein is for the purpose of describing particular embodiments of the invention only, and is not intended to be limiting.

Although a number of methods and materials similar or equivalent to those described herein can be used in the practice of the present invention, the preferred materials and methods are described herein.

Central to the present invention is the discovery of a new *Helicobacter* species isolated from swine with gastritis/ulcer disease. This organism, named, *H. cerdo* (Hc) by the inventors herein, produces gastric disease in young piglets that is similar to the Hp-associated active gastritis in humans. Moreover, immunogens from *H. cerdo* provide protection against subsequent challenge with *Helicobacter* species and provide diagnostic reagents for detecting *Helicobacter* infection, such as *H. cerdo* infection, in vertebrate subjects such as swine. *H. cerdo* vaccines can be used against a wide range of *Helicobacter* isolates. Moreover, the vaccines are safe, economic, have an indefinite shelf life and can be efficiently administered parenterally.

In order to further an understanding of the invention, a more detailed discussion is provided below regarding *H. cerdo* immunogens, as well as various uses thereof.

### H. cerdo immunogens

The *H. cerdo* immunogens for use in vaccine and diagnostic compositions can be produced using a variety of techniques. For example, the immunogens can be obtained directly from *H. cerdo* bacteria that have been isolated from swine using techniques well known in the art and described in the examples herein. Generally, *H*. *cerdo* bacteria are obtained from young, weanling swine, typically three weeks to eight weeks of age, more typically five to six weeks of age, before the onset of ulcer disease. The presence of the bacterium can be detected as described in the examples, e.g., by microscopic examination, as well as by detecting the activity of the enzyme urease and/or catalase. For example, urease catalyzes the conversion of urea to ammonium causing an increase in the pH of the culture medium. The pH change can be detected by a color change to the medium due to the presence of a pH sensitive indicator. See, e.g., U.S. Patent No. 5,498,528.

*H. cerdo* immunogen from the bacteria can be provided in a lysate that can be obtained using methods well known in the art. Generally, such methods entail extracting proteins from *H. cerdo* bacteria using such techniques as sonication or ultrasonication; agitation; liquid or solid extrusion; heat treatment; freeze-thaw techniques; explosive decompression; osmotic shock; proteolytic digestion such as treatment with lytic enzymes including proteases such as pepsin, trypsin, neuraminidase and lysozyme; alkali treatment; pressure disintegration; the use of detergents and solvents such as bile salts, sodium dodecylsulphate, TRITON, NP40 and CHAPS; fractionation, and the like. The particular technique used to disrupt the cells is largely a matter of choice and will depend on the culture conditions and any pre-treatment used. Following disruption of the cells, cellular debris can be removed, generally by centrifugation and/or dialysis.

The immunogens present in such lysates can be further purified if desired, using standard purification techniques such as but not limited to, column chromatography, ion-exchange chromatography, size-exclusion chromatography, electrophoresis, HPLC, immunoadsorbent techniques, affinity chromatography, immunoprecipitation, and the like. See, e.g., International Publication No. WO 96/12965, published 2 May 1996, for a description of the purification of several antigens from *H. pylori.* Such techniques are also useful for purifying antigens from *H. cerdo.*

The *H. cerdo* immunogens can also be generated using recombinant methods, well known in the art. In this regard, oligonucleotide probes can be devised based on the sequences of the *H. cerdo* and/or *H. pylori* genome and used to probe genomic or cDNA libraries for *H. cerdo* genes encoding for the antigens useful in the present invention. The genes can then be further isolated using standard techniques and, if desired, restriction enzymes employed to mutate the gene at desired portions of the full-length sequence.

Similarly, *H. cerdo* genes can be isolated directly from bacterial cells using known techniques, such as phenol extraction, and the sequence can be further manipulated to produce any desired alterations. See, e.g., Sambrook et al., *supra,* for a description of techniques used to obtain and isolate DNA. Finally, the genes encoding the *H. cerdo* immunogens can be produced synthetically, based on the known sequences. The nucleotide sequence can be designed with the appropriate codons for the particular amino acid sequence desired. In general, one will select preferred codons for the intended host in which the sequence will be expressed. The complete sequence is generally assembled from overlapping oligonucleotides prepared by standard methods and assembled into a complete coding sequence. See, e.g., Edge, Nature (1981) 292:756; Nambair et al., Science (1984) 223:1299; Jay et al., J. Biol. Chem. (1984) 259:6311.

Once coding sequences for the desired polypeptides have been isolated or synthesized, they can be cloned into any suitable vector or replicon for expression in a variety of systems, including insect, mammalian, bacterial, viral and yeast expression systems, all well known in the art. In particular, host cells are transformed with expression vectors which include control sequences operably linked to the desired coding sequence. The control sequences will be compatible with the particular host cell used. It is often desirable that the polypeptides prepared using the above systems be fusion polypeptides. As with nonfusion proteins, these proteins may be expressed intracellularly or may be secreted from the cell into the growth medium.

Furthermore, plasmids can be constructed which include a chimeric gene sequence, encoding e.g., multiple *H. cerdo* antigens. The gene sequences can be present in a dicistronic gene configuration. Additional control elements can be situated between the various genes for efficient translation of RNA from the distal coding region. Alternatively, a chimeric transcription unit having a single open reading frame encoding the multiple antigens can also be constructed. Either a fusion can be made to allow for the synthesis of a chimeric protein or alternatively, protein processing signals can be engineered to provide cleavage by a protease such as a signal peptidase, thus allowing liberation of the two or more proteins derived from translation of the template RNA. The processing protease may also be expressed in this system either independently or as part of a chimera with the antigen and/or cytokine coding region(s). The protease itself can be both a processing enzyme and a vaccine antigen.

Depending on the expression system and host selected, the immunogens of the present invention are produced by growing host cells transformed by an expression vector under conditions whereby the immunogen of interest is expressed. The immunogen is then isolated from the host cells and purified. If the expression system provides for secretion of the immunogen, the immunogen can be purified directly from the media. If the immunogen is not secreted, it is isolated from cell lysates. The selection of the appropriate growth conditions and recovery methods are within the skill of the art.

The *H. cerdo* immunogens may also be produced by chemical synthesis such as by solid phase or solution peptide synthesis, using methods known to those skilled in the art. Chemical synthesis of peptides may be preferable if the antigen in question is relatively small. See, e.g., J. M. Stewart and J. D. Young, Solid Phase Peptide Synthesis, 2nd Ed., Pierce Chemical Co., Rockford, IL (1984) and G. Barany and R. B. Merrifield, The Peptides: Analysis, Synthesis, Biology, editors E. Gross and J. Meienhofer, Vol. 2, Academic Press, New York, (1980), pp. 3-254, for solid phase peptide synthesis techniques; and M. Bodansky, Principle of Peptide Synthesis, Springer-Verlag, Berlin (1984) and E. Gross and J. Meienhofer, Eds., The Peptides: Analysis, Synthesis, Biology, supra, Vol. 1, for classical solution synthesis.

The *H. cerdo* immunogens, including *H. cerdo* lysates, can be used to produce antibodies, both polyclonal and monoclonal. If polyclonal antibodies are desired, a selected mammal, (e.g., mouse, rabbit, goat, horse, etc.) is immunized with an immunogen of the present invention, or its fragment, or a mutated immunogen. Serum from the immunized animal is collected and treated according to known procedures. See, e.g., Jurgens et al. (1985) J. Chrom. 348:363-370. If serum containing polyclonal antibodies is used, the polyclonal antibodies can be purified by immunoaffinity chromatography, using known procedures.

Monoclonal antibodies to the *H. cerdo* immunogens, can also be readily produced by one skilled in the art. The general methodology for making monoclonal antibodies by using hybridoma technology is well known. Immortal antibody-producing cell lines can be created by cell fusion, and also by other techniques such as direct transformation of B lymphocytes with oncogenic DNA, or transfection with Epstein-Barr virus. See, e.g., M. Schreier et al., Hybridoma Techniques (1980); Hammerling et al., Monoclonal Antibodies and T-cell Hybridomas (1981); Kennett et al., Monoclonal Antibodies (1980); see also U.S. Patent Nos. 4,341,761; 4,399,121; 4,427,783; 4,444,887; 4,452,570; 4,466,917; 4,472,500,4,491,632; and 4,493,890. Panels of monoclonal antibodies produced against the *H. cerdo* immunogen of interest, or fragment thereof, can be screened for various properties; i.e., for isotype, epitope, affinity, etc. Monoclonal antibodies are useful in purification, using immunoaffinity techniques, of the individual antigens which they are directed against. Both polyclonal and monoclonal antibodies can also be used for passive immunization or can be combined with subunit vaccine preparations to enhance the immune response.

### H. cerdo Formulations and Administration

The *H. cerdo* immunogens of the present invention, including the *H. cerdo* lysates, can be formulated into compositions, such as vaccine or diagnostic compositions, either alone or in combination with other antigens, for use in immunizing subjects as described below. Methods of preparing such formulations are described in, e.g., Remington's Pharmaceutical Sciences, Mack Publishing Company, Easton, Pennsylvania, 18 Edition, 1990. Typically, the vaccines of the present invention are prepared as injectables, either as liquid solutions or suspensions. Solid forms suitable for solution in or suspension in liquid vehicles prior to injection may also be prepared. The preparation may also be emulsified or the active ingredient encapsulated in liposome vehicles. The active immunogenic ingredient is generally mixed with a compatible pharmaceutical vehicle, such as, for example, water, saline, dextrose, glycerol, ethanol, or the like, and combinations thereof. In addition, if desired, the vehicle may contain minor amounts of auxiliary substances such as wetting or emulsifying agents and pH buffering agents.

Adjuvants which enhance the effectiveness of the vaccine may also be added to the formulation. Adjuvants may include for example, muramyl dipeptides, avridine, aluminum hydroxide, alum, Freund's adjuvant, incomplete Freund's adjuvant (ICFA), dimethyldioctadecyl ammonium bromide (DDA), oils, oil-in-water emulsions, saponins, cytokines, and other substances known in the art. Such adjuvants are well known and commercially available from a number of sources, e.g., Difco, Pfizer Animal Health, Newport Laboratories, etc.

The *H. cerdo* immunogens may also be linked to a carrier in order to increase the immunogenicity thereof. Suitable carriers include large, slowly metabolized macromolecules such as proteins, including serum albumins, keyhole limpet hemocyanin, immunoglobulin molecules, thyroglobulin, ovalbumin, and other proteins well known to those skilled in the art; polysaccharides, such as sepharose, agarose, cellulose, cellulose beads and the like; polymeric amino acids such as polyglutamic acid, polylysine, and the like; amino acid copolymers; and inactive virus particles.

The *H. cerdo* immunogens may be used in their native form or their functional group content may be modified by, for example, succinylation of lysine residues or reaction with Cys-thiolactone. A sulfhydryl group may also be incorporated into the carrier (or antigen) by, for example, reaction of amino functions with 2-iminothiolane or the N-hydroxysuccinimide ester of 3-(4-dithiopyridyl propionate. Suitable carriers may also be modified to incorporate spacer arms (such as hexamethylene diamine or other bifunctional molecules of similar size) for attachment of peptides.

Furthermore, the *H. cerdo* immunogens may be formulated into vaccine compositions in either neutral or salt forms. Pharmaceutically acceptable salts include the acid addition salts (formed with the free amino groups of the active polypeptides) and which are formed with inorganic acids such as, for example, hydrochloric or phosphoric acids, or such organic acids as acetic, oxalic, tartaric, mandelic, and the like. Salts formed from free carboxyl groups may also be derived from inorganic bases such as, for example, sodium, potassium, ammonium, calcium, or ferric hydroxides, and such organic bases as isopropylamine, trimethylamine, 2-ethylamino ethanol, histidine, procaine, and the like.

Vaccine formulations will contain a "therapeutically effective amount" of the active ingredient, that is, an amount capable of eliciting an immune response in a subject to which the composition is administered. In the treatment and prevention of *Helicobacter* infection, a "therapeutically effective amount" is readily determined by one skilled in the art using standard tests. The *H. cerdo* immunogens will typically range from about 1% to about 95% (w/w) of the composition, or even higher or lower if appropriate. With the present vaccine formulations, .1 to 500 mg of active ingredient per ml, preferably 1 to 100 mg/ml, more preferably 10 to 50 mg/ml, such as 20...25...30...35...40, etc., or any number within these stated ranges, of injected solution should be adequate to raise an immunological response when a dose of .25 to 3 ml per animal is administered.

To immunize a subject, the vaccine is generally administered parenterally, usually by intramuscular injection. Other modes of administration, however, such as subcutaneous, intraperitoneal and intravenous injection, are also acceptable. The quantity to be administered depends on the animal to be treated, the capacity of the animal's immune system to synthesize antibodies, and the degree of protection desired. Effective dosages can be readily established by one of ordinary skill in the art through routine trials establishing dose response curves. The subject is immunized by administration of the vaccine in at least one dose, and preferably two or more doses. Moreover, the animal may be administered as many doses as is required to maintain a state of immunity to infection.

Additional vaccine formulations which are suitable for other modes of administration include suppositories and, in some cases, aerosol, intranasal, oral formulations, and sustained release formulations. For suppositories, the vehicle composition will include traditional binders and carriers, such as, polyalkaline glycols, or triglycerides. Such suppositories may be formed from mixtures containing the active ingredient in the range of about 0.5% to about 10% (w/w), preferably about 1% to about 2%. Oral vehicles include such normally employed excipients as, for example, pharmaceutical grades of mannitol, lactose, starch, magnesium, stearate, sodium saccharin cellulose, magnesium carbonate, and the like. These oral vaccine compositions may be taken in the form of solutions, suspensions, tablets, pills, capsules, sustained release formulations, or powders, and contain from about 10% to about 95% of the active ingredient, preferably about 25% to about 70%.

Intranasal formulations will usually include vehicles that neither cause irritation to the nasal mucosa nor significantly disturb ciliary function. Diluents such as water, aqueous saline or other known substances can be employed with the subject invention. The nasal formulations may also contain preservatives such as, but not limited to, chlorobutanol and benzalkonium chloride. A surfactant may be present to enhance absorption of the subject proteins by the nasal mucosa.

Controlled or sustained release formulations are made by incorporating the protein into carriers or vehicles such as liposomes, nonresorbable impermeable polymers such as ethylenevinyl acetate copolymers and Hytrel copolymers, swellable polymers such as hydrogels, or resorbable polymers such as collagen and certain polyacids or polyesters such as those used to make resorbable sutures. The *H. cerdo* immunogens can also be delivered using implanted mini-pumps, well known in the art.

The *H. cerdo* immunogens of the instant invention can also be administered via a carrier virus which expresses the same. Carrier viruses which will find use with the instant invention include but are not limited to the vaccinia and other pox viruses, adenovirus, and herpes virus. By way of example, vaccinia virus recombinants expressing the novel proteins can be constructed as follows. The DNA encoding the particular protein is first inserted into an appropriate vector so that it is adjacent to a vaccinia promoter and flanking vaccinia DNA sequences, such as the sequence encoding thymidine kinase (TK). This vector is then used to transfect cells which are simultaneously infected with vaccinia. Homologous recombination serves to insert the vaccinia promoter plus the gene encoding the instant protein into the viral genome. The resulting TK⁻recombinant can be selected by culturing the cells in the presence of 5-bromodeoxyuridine and picking viral plaques resistant thereto.

An alternative route of administration involves gene therapy or nucleic acid immunization. Thus, nucleotide sequences (and accompanying regulatory elements) encoding the subject *H. cerdo* immunogens can be administered directly to a subject for *in vivo* translation thereof. Alternatively, gene transfer can be accomplished by transfecting the subject's cells or tissues *ex vivo* and reintroducing the transformed material into the host. DNA can be directly introduced into the host organism, i.e., by injection (see International Publication No. WO/90/11092; and Wolff et al. (1990) Science 247:1465-1468). Liposome-mediated gene transfer can also be accomplished using known methods. See, e.g., Hazinski et al. (1991) Am. J. Respir. Cell Mol. Biol. 4:206-209; Brigham et al. (1989) Am. J. Med. Sci. 298:278-281; Canonico et al. (1991) Clin. Res. 39:219A; and Nabel et al. (1990) Science 1990) 249:1285-1288. Targeting agents, such as antibodies directed against surface antigens expressed on specific cell types, can be covalently conjugated to the liposomal surface so that the nucleic acid can be delivered to specific tissues and cells susceptible to infection.

The compositions of the present invention can be administered prior to, subsequent to or concurrently with traditional antimicrobial agents used to treat *Helicobacter* disease, such as but not limited to bismuth subsalicylate, metronidazole, amoxicillin, omeprazole, clarithromycin, ciprofloxacin, erythromycin, tetracycline, nitrofurantoin, ranitidine, omeprazole, and the like. One particularly preferred method of treatment is to first administer conventional antibiotics as described above followed by vaccination with the compositions of the present invention once the *Helicobacter* infection has cleared.

### Diagnostics

The *H. cerdo* immunogens, including *H. cerdo* lysates, can also be used as diagnostics to detect the presence of reactive antibodies directed against the bacterium in a biological sample. Furthermore, the immunogens can be used to monitor the course of antibiotic therapy by comparing results obtained at the outset of therapy to those obtained during and after a course of treatment. For example, the presence of antibodies reactive with the *H. cerdo* antigens can be detected using standard electrophoretic and immunodiagnostic techniques, including immunoassays such as competition, direct reaction, or sandwich type assays. Such assays include, but are not limited to, Western blots; agglutination tests; enzyme-labeled and mediated immunoassays, such as ELISAs; biotin/avidin type assays; radioimmunoassays; immunoelectrophoresis; immunoprecipitation, etc. The reactions generally include revealing labels such as fluorescent, chemiluminescent, radioactive, enzymatic labels or dye molecules, or other methods for detecting the formation of a complex between the antigen and the antibody or antibodies reacted therewith.

The aforementioned assays generally involve separation of unbound antibody in a liquid phase from a solid phase support to which antigen-antibody complexes are bound. Solid supports which can be used in the practice of the invention include substrates such as nitrocellulose (e.g., in membrane or microtiter well form); polyvinylchloride (e.g., sheets or microtiter wells); polystyrene latex (e.g, beads or microtiter plates); polyvinylidine fluoride; diazotized paper; nylon membranes; activated beads, magnetically responsive beads, and the like.

Typically, a solid support is first reacted with a solid phase component (e.g., one or more *H. cerdo* antigens, such as an *H. cerdo* lysate produced by proteolytic digestion of *H. cerdo* bacteria) under suitable binding conditions such that the component is sufficiently immobilized to the support. Sometimes, immobilization of the antigen to the support can be enhanced by first coupling the antigen to a protein with better binding properties. Suitable coupling proteins include, but are not limited to, macromolecules such as serum albumins including bovine serum albumin (BSA), keyhole limpet hemocyanin, immunoglobulin molecules, thyroglobulin, ovalbumin, and other proteins well known to those skilled in the art. Other molecules that can be used to bind the antigens to the support include polysaccharides, polylactic acids, polyglycolic acids, polymeric amino acids, amino acid copolymers, and the like. Such molecules and methods of coupling these molecules to the antigens, are well known to those of ordinary skill in the art. See, e.g., Brinkley, M.A., Bioconjugate Chem. (1992) 3:2-13; Hashida et al., J. Appl. Biochem. (1984) 6:56-63; and Anjaneyulu and Staros, International J. of Peptide and Protein Res. (1987) 30:117-124.

After reacting the solid support with the solid phase component, any non-immobilized solid-phase components are removed from the support by washing, and the support-bound component is then contacted with a biological sample suspected of containing ligand moieties (e.g., antibodies toward the immobilized antigens) under suitable binding conditions. After washing to remove any non-bound ligand, a secondary binder moiety is added under suitable binding conditions, where the secondary binder is capable of associating selectively with the bound ligand. The presence of the secondary binder can then be detected using techniques well known in the art.

More particularly, an ELISA method can be used, where the wells of a microtiter plate are coated with the *H. cerdo* antigen(s). A biological sample containing or suspected of containing anti-H. *cerdo* immunoglobulin molecules is then added to the coated wells. In assays where it is desired to use one microtiter plate, a selected number of wells can be coated with, e.g., a first antigen moiety, a different set of wells coated with a second antigen moiety, and so on. In the alternative, a series of ELISAs can be run in tandem. After a period of incubation sufficient to allow antibody binding to the immobilized antigens, the plate(s) can be washed to remove unbound moieties and a detectably labeled secondary binding molecule added. The secondary binding molecule is allowed to react with any captured sample antibodies, the plate washed and the presence of the secondary binding molecule detected using methods well known in the art.

Thus, in one particular embodiment, the presence of bound anti-*H*. *cerdo* antigen ligands from a biological sample can be readily detected using a secondary binder comprising an antibody directed against the antibody ligands. A number useful immunoglobulin (Ig) molecules are known in the art and commercially available. Ig molecules for use herein will preferably be of the IgG or IgA type, however, IgM may also be appropriate in some instances. The Ig molecules can be readily conjugated to a detectable enzyme label, such as horseradish peroxidase, glucose oxidase, Beta-galactosidase, alkaline phosphatase and urease, among others, using methods known to those of skill in the art. An appropriate enzyme substrate is then used to generate a detectable signal. In other related embodiments, competitive-type ELISA techniques can be practiced using methods known to those skilled in the art.

Assays can also be conducted in solution, such that the bacterial proteins and antibodies specific for those bacterial proteins form complexes under precipitating conditions. In one particular embodiment, the *H. cerdo* antigen(s) can be attached to a solid phase particle (e.g., an agarose bead or the like) using coupling techniques known in the art, such as by direct chemical or indirect coupling. The antigen-coated particle is then contacted under suitable binding conditions with a biological sample suspected of containing antibodies for *H. cerdo.* Cross-linking between bound antibodies causes the formation of particle-antigen-antibody complex aggregates which can be precipitated and separated from the sample using washing and/or centrifugation. The reaction mixture can be analyzed to determine the presence or absence of antibody-antigen complexes using any of a number of standard methods, such as those immunodiagnostic methods described above.

In yet a further embodiment, an immunoaffinity matrix can be provided, wherein a polyclonal population of antibodies from a biological sample suspected of containing anti-*H*. *cerdo* antibodies is immobilized to a substrate. In this regard, an initial affinity purification of the sample can be carried out using immobilized antigens. The resultant sample preparation will thus only contain anti-*H*. *cerdo* moieties, avoiding potential nonspecific binding properties in the affinity support. A number of methods of immobilizing immunoglobulins (either intact or in specific fragments) at high yield and having good retention of antigen binding activity, are known in the art. Not being limited by any particular method, immobilized protein A or protein G can be used to immobilize immunoglobulins.

Accordingly, once the immunoglobulin molecules have been immobilized to provide an immunoaffinity matrix, the *H. cerdo* antigens, having separate and distinct labels, are contacted with the bound antibodies under suitable binding conditions. After any non-specifically bound antigen has been washed from the immunoaffinity support, the presence of bound antigen can be determined by assaying for each specific label using methods known in the art.

The above-described assay reagents, including the *H. cerdo* immonogens (such as an *H. cerdo* lysate), optionally immobilized on a solid support, can be provided in kits, with suitable instructions and other necessary reagents, in order to conduct immunoassays as described above. The kit can also contain, depending on the particular immunoassay used, suitable labels and other packaged reagents and materials (i.e. wash buffers and the like). Standard immunoassays, such as those described above, can be conducted using these kits.

### 3. EXPERIMENTAL

Efforts have been made to ensure accuracy with respect to numbers used (e.g., amounts, temperatures, etc.), but some experimental error and deviation should, of course, be allowed for.

### Example 1

### Isolation of H. cerdo from Porcine Gastric Mucosa

Bacteria were recovered from porcine gastric mucosa under microaerophilic conditions as follows. Stomachs were removed from young swine and opened by incision along the greater and lesser curvatures. Contents were removed and the mucosa was rinsed with sterile saline washes. Mucosal strips from the glandular cardia of the lesser curvature and mucosal antrum, 5 x 20 mm, (less the muscularis), were removed by sterile dissection and suspended in 5 ml of Brucella broth (Difco) supplemented with 10% fetal bovine serum (B-FBS) and the strip was placed in sterile 7.0 ml glass ten Broeck tissue grinders. The tissues were ground 10 times and 10-fold serial dilutions (10¹⁰ to 10⁻⁴) were made in B-FBS. 1/10 ml of each dilution was plated onto agar plates containing either Skirrow's medium or TSAII (trypticase soy agar with 5% sheep blood). Plates were incubated in a humid microaerobic environment for 3-4 days. Suspect *Helicobacter* species colonies (small pinpoint translucent and non-hemolytic) were identified and sub-passed onto fresh agar plates as above.

Aliquots of each suspect isolate were stained by the Gram's stain method and tested for urease activity (placement of a cotton swab containing the organisms into B-FBS containing urea and pH indicator) and into a solution of 1% (v/v) hydrogen peroxide in sterile distilled water. Microbes which were Gram negative short curved rods which were urease- and catalase-positive were considered to be *Helicobacter* species.

On the basis of location (stomach), morphology (Gram negative, short, curved "gull-wing-like" rods), urease activity and cross-reactivity with an anti-Hp reagent, the bacterium isolated was assigned to the genus *Helicobacter* and named *H. cerdo* (Spanish for "pork").

*H. cerdo* is distinct from, but antigenically related to Hp, and the larger spiral organism, *Candidatus Helicobacter suis* (Degroote et al. (2000) J. Clin. Microbiol. 38:1131-1135), another *Helicobacter* species that is found in normal swine and swine with gastritits and is therefore thought to be a nonpathogenic commensal organism.

### Example 2

### Infection and Recovery of H. cerdo from Experimentally Infected Swine

Three gnotobiotic piglets were orally infected with *H. cerdo* at three days of age and terminated at 35 days of age. A procedure similar to that detailed above was used to recover gastric bacteria from the experimentally infected swine. For this, one-half of the stomach was sterilely removed and placed into sterile pre-weighed 100 mm³ petri dishes. 5 ml of B-FBS was added and the mucosa was separated from the gastric muscularis by blunt dissection and scraping with sterile instruments. The muscularis was removed and the petri plates containing the recovered mucosa were weighed again. The mucosa and B-FBS were removed and placed into sterile 7.0 ml glass ten Broeck tissue grinders and ground as above. 10-fold serial dilutions of the homogenate were made in B-FBS and 1/10 ml of each dilution was plated in duplicate onto TSAII or blood agar plates. Plates were incubated in a humid microaerobic environment for 3-4 days.

Suspect *Helicobacter* species colonies (small pinpoint translucent and non-hemolytic) were identified on each plate dilution. Discrete colonies were counted on the plate/dilution containing between 30 and 300 bacterial colonies. To determine bacterial colony forming units (cfu) per gram of gastric mucosa, the number of colonies counted between the two dilutions was averaged (total colonies counted divided by 2) and multiplied by the dilution factor (10⁻⁰ to 10⁻⁴), by 5 (for the initial dilution in B-FBS), times 10 (for the initial dilution) to arrive at the total cfu recovered. The total cfu was divided by the weight of gastric mucosa and the resultant number was the bacterial cfu/gram of gastric mucosa.

Tables 1-4 summarize the gross observations (Table 1), histopathologic changes (Table 2), extra-gastric histopathologic findings (Table 3) and microbiologic findings (Table 4) in the infected pigs. All of the tested pigs (3/3) were culture and W/S positive in the stomach. 3/3 pigs displayed gastroesophageal ulceration (GEU) in nonglandular cardia; 2/3 showed healed antral microulcers; and 3/3 displayed lyphofollicular antral gastritis. Thus, *H. cerdo* colonized the gastric mucosa of the swine. Additionally, *H. cerdo* infection was strongly associated with gastric and duodenal ulcer disease and produced a persistent gastric bacterial infection of swine analogous to *H. pylori* in humans.

**Table 1. A summary of gross observations in gnotobiotic piglets infected with H. cerdo and terminated at 35 days of age.**

| Group & and/or Piglet No. | Wt. (Gms) | Gender (M/F) | Excess Mucus | Lymphoid Follicles | Submucosal Edema | Skin 24hr | Tests^{a} 48 hr | Ulcers Erosions |
|---|---|---|---|---|---|---|---|---|
| 02-2662 | 2650 | M | 1^{b} | 2 | 2 | -^{c} | slight | GEU, red lessercurvature |
| 02-2663 | 2700 | F | 2 | 2 | 0 | - | - | GEU, lesser curvature, possible |
| | | | | | | | | |
| | ulcer in fundus | | | | | | | |
| 02-2664 | 2960 | F | 2 | 3 | 3 | - | - | GEU, lesser curvature, possible |
| | | | | | | | | |
| | ulcer in antrum | | | | | | | |

| | | | | | | | | |
|---|---|---|---|---|---|---|---|---|
| ^{a}Skin test antigen consisted of *Helicobacter pylori* preparation, (10.0 ug 26695 clarified sonicate in 0.1 ml PBS). ^{b}Visually scored as 0 = no change from normal; 1 = minimal change; 2 = moderate change; and 3 = severe change ^{c}Skin test responses scored as negative (-) or positive (+) with further description. | | | | | | | | |

**Table 2. A summary of histopathologic changes in the stomachs of gnotobiotic piglets infected with H. cerdo and terminated at 35 days of age.**

| | Anatomical Region of the Stomach | | | | | | | |
|---|---|---|---|---|---|---|---|---|
| Group And Piglet ID number | | Cardia | | Fundus | | Antrum | | Pylorus |
| | | H/E | W/Sa | H/E | W/S | H/E | W/S | H/E |
| | W/S | | | | | | | |
| 02-2662 | | 3^{b} | +^{c} | 1 | X^{d} | 2 | X | 0 |
| X | | | GEU^{e} | | | possible healed | | |
| | | | | | | | micro-ulcer | |
| | | | | | | | | |
| | | | | | 02-2663 | 3 | +/- | 0 |
| X | 1 | X | | 1 | X | | | |
| | | | | | | | | |
| 02-2664 | | 3 | + | 0 | X | 3 | X | 0 |
| X | | | | | | | | |
| | | | | | | possible healed | | |
| | | | | | | micro-ulcer | | |

| | | | | | | | | |
|---|---|---|---|---|---|---|---|---|
| ^{a}H/E = hematoxylin and eosin stain; W/S = Warthin-Starry stain ^{b} Subjectively scored as 0 = no change from normal (no inflammation); 1 = minimal change from normal; 2 = moderate change from normal; and 3 = severe change from normal. ^{c} Scored as (+) for small curved extracellular micro-organisms present on the gastric luminal surface of the sections or (-): no microbes seen. ^{d} X - The W/S stains are of poor quality and must be repeated before a determination of the presence of organisms can be determined. ^{e} GEU: gastroesophageal ulceration in the nonglandular cardia and adjacent glandular mucosa of the lesser curvature of the stomach. | | | | | | | | |

**Table 3. A summary of extra-gastric histopathologic findings in gnotobiotic piglets infected with H. cerdo and terminated at 35 days of age.**

| Group and Piglet ID number | Anatomical Region of the Gastrointestinal Tract | | | | | Skin test sites (ear) | | |
|---|---|---|---|---|---|---|---|---|
| | esophagus | duodenum | jejunum | ileum | colon | gastric | 24 hr | 48 hr |
| | | | | | lymph nodes | | | |
| 02-2662 | 0^{a} | 0 | 0 | reactive patches | 0 | reactive Peyer's | 0^{b} | 1 |
| | | | | | | | hyperplasia mononuclear cell infiltrates | |
| | | | | | | | | |
| | | | | | | | | |
| | | | | | | | | |
| 02-2663 | 0 | villous atrophy | 0 | reactive Peyer's patches | 0 | ndc PMNs & mononuclears | 1 | 0 |
| 02-2664 | 0 | 0 | 0 | reactive Peyer's | reactive follicles | reactive lymphoid patches | 0 | 1 |
| | | | | | | | | hyperplasia mononuclearcell infiltrates |

| | | | | | | | | |
|---|---|---|---|---|---|---|---|---|
| ^{a}Subjectively scored as 0 = no change from normal (no inflammation); 1 = minimal change from normal; 2 = moderate change from normal; and 3 = severe change from normal on H/E-stained sections. ^{b}Skin test sites (ear) scored as 0 (no inflammatory cell infiltrate) or 1 (modest inflammatory cell infiltrates ^{c}nd: not done | | | | | | | | |

**Table 4. A summary of microbiologic findings in gnotobiotic piglets infected with H. cerdo and terminated at 35 days of age.**

| Group and Piglet No. | *H. cerdo* at Termination (PID 35) | | | | Other Microbial Contaminants |
|---|---|---|---|---|---|
| | cfu/gm (x106) | Urease | Cata | Oxi | |
| 02-2662 | 5.54 x 105 | + | + | + | none |
| 02-2663 | + (re-streaks) | + | + | + | none |
| 02-2664 | 5.52 x 106 | + | + | + | none |

### Example 3

### Prevention of H. cerdo Infection using an H. cerdo Lysate

An *H. cerdo* vaccine was prepared using proteolytic digestion to produce an *H. cerdo* lysate, according to a method similar to the digestion protocol described in Waters et al. (2000) Vaccine 18:711-719. In particular, suspensions of *H. cerdo* bacteria propagated in liquid cultures of B-FBS under microaerophilic conditions were allowed to reach approximately 10⁹ bacteria per ml. The bacteria were recovered by centrifugation (2000-3000 x g) for 10 minutes. The spent supernatant was discarded and the bacterial pellet was resuspended in a minimal amount of Dulbecco's phosphate-buffered saline, transferred to a plastic cryo vial and frozen at-70 degrees C. While frozen, the bacterial pellet was lyophilized in a centrifugal evaporator apparatus (speed vac). Lyophilized bacterial pellets were pooled and weighed. For bacterial digestion, pepsin (Sigma, St. Louis, MO) at a concentration of 1.0 µg/ml was prepared by dilution into 10 mM HCl, pH 1.9-2.2. 1 µg of pepsin was incubated with 1 mg of lyophilized bacteria for 24-25 hours at 37 degrees C on a magnetic stirrer. After completion of digestion, the digest was aliquoted, labeled and frozen at -70 degrees C until use.

The *H. cerdo* lysate was formulated into a vaccine composition and used to vaccinate gnotobiotic pigs as follows. The lysate was diluted to 24-25 mg/ml in Dulbecco's phosphate-buffered saline and mixed with 1 ml of adjuvant. The vaccine was emulsified in adjuvant and .5 ml of the mixture was injected into the dorsal axillas and hips of each piglet. Each piglet received 3 injections at 3, 10 and 17 days of age (see, Table 5).

The results indicated significant reduction in pathogen loads and disease sparing in vaccinated pigs, demonstrating the efficacy of this immunoprophylactic approach. In particular, as seen in the tables herein, *H. cerdo* infects piglets and persistently colonizes gastric mucosa and segments of the proximal small intestine. *H. cerdo* is associated with gastric ulcer disease. Homologous, parenterally administered vaccine protected against subsequent oral challenge with infection by *H*. *cerdo.*

**Table 5. Experimental Design and Evaluation**

| Piglet and Group No. | Vaccinate at 3, 10 and 17 days of age with: Infect with *H. cerdo* on day 21: | | |
|---|---|---|---|
| | *H. pylori* digest | *H. cerdo* digest | 24 days of age |
| Isolator no 1 | | | |
| A (n=2) | yes | - | yes |
| B (n=2) | - | yes | yes |
| C (n=2) | - | - | yes |

| | | | |
|---|---|---|---|
| 1. Piglets were terminated approximately 2 weeks after challenge with *H. cerdo* (35 days of age). 2. One-half of the stomach was removed, weighed, mucosa scraped free of the muscularis and weighed again. A 10% (w/v) homogenate was made and quantitative re-isolation of organisms was determined by titration onto microtiter plates. Organisms were confirmed to be of *Helicobacter spp* by urease, catalase assays, Gram's stain and colony morphology. 3. The remaining one-half stomach was examined for histologic evidence of disease by standard methods. 4. For the two piglets of group C, sterile samples of esophagus, duodenum, jejenum, ileum, spiral colon, descending colon and terminal colon was also cultured for the presence of organisms (positive or negative, nonquantitative), to determine if H. suis is a stomach-specific pathogen of swine as H. pylori is in experimentally infected gnotobiotic swine and also in humans. | | | |

**Table 6. A summary of gross observations in gnotobiotic piglets vaccinated with protease digests, infected with H. cerdo and terminated at 35 days of age.**

| Group & Piglet No. | Wt. (Gms) | Gender (M/F) | Excess Mucus | Lymphoid Follicles | Submucosal Edema | Ulcers and/or Erosions |
|---|---|---|---|---|---|---|
| Vaccinated with *H. pylori* digest and infected with *H. cerdo* | | | | | | |
| 02-3481 | 2750 | F | 0^{a} | +/- | 0 | none |
| 02-3482 | 3400 | M | 1 | 0 | 0 | none |

| Vaccinated with *H. cerdo* digest and infected with *H. cerdo* | | | | | | |
|---|---|---|---|---|---|---|
| 02-3484 | 2840 | M | 1 | 1 | 1 | GEU - mild |
| 02-3485 | 3410 | M | 1 | 2 | 1 | possible GEU & ulcer |

| Unvaccinated and infected with *H. cerdo* | | | | | | |
|---|---|---|---|---|---|---|
| 02-3483 | 2970 | F | 1 | 3 | 1 | massive GEU, hemorrhage |
| 02-3486 | 3520 | M | 1 | 3 | 1 | small GEU |

| | | | | | | |
|---|---|---|---|---|---|---|
| ^{a} Visually scored as 0 = no change from normal; +/- = possible change from normal; 1 = minimal change; 2 = moderate change; and 3 = severe change | | | | | | |

Isotype-specific ELISAs were performed in order to detect serum antibodies directed against *Helicobacter* species antigen in sera from *H. cerdo-* and *H*. *pylori-*infected pigs as described in Krakowka et al. (1987) Inflect. Immun. 55:2789-2796; Krakowka et al. (1996) Vet. Immunol. Immunopathol. 55:2789-2796; and Eaton et al. (1992) Gastroenterol. 103:1580-1586. The vaccine in saline alone without adjuvant or combined with the adjuvants described further below stimulated IgG isotype-specific antibodies. Moreover, sera from *H. cerdo*-infected and *H*. *pylori*-infected pigs cross-reacted in the ELISA when either bacterial antigen was used. See, Tables 7-9.

**Table 7. ELISA (IgG) serum antibody responses to lysates of Helicobacter species in gnotobiotic piglets vaccinated three times with H. pylori proteolytic digest, orally infected with a suboptimal amount of H. pylori and terminated at 24 days of age.**

| Group & Piglet | *Helicobacter pylori* antigen: | | | *Helicobacter cerdo* antigen: | | |
|---|---|---|---|---|---|---|
| | Pre-vaccinatior | Pre-challenge | Terminal | Pre-vaccination | Pre-challenge | Terminal |
| Group A: Vaccinated three times with Protease Digest in Squalene and challenged with *H pylori* | | | | | | |
| 01-4161 | --- | 0.86 | 1.02 | --- | 0.74 | 0.81 |
| 01-4162 | --- | 1.07 | 1.35 | --- | 1.18 | 1.45 |
| 01-4163 | --- | 1.02 | 1.25 | --- | 0.92 | 1.05 |

| Group B: Vaccinated three times with saline alone and challenged with *H pylori* | | | | | | |
|---|---|---|---|---|---|---|
| 01-4164 | --- | --- | --- | --- | --- | --- |
| 01-4165 | --- | --- | --- | --- | --- | --- |
| 01-4166 | --- | --- | --- | --- | --- | --- |

| Group C: Vaccinated three times with Protease Digest in saline and challenged with *H pylori* | | | | | | |
|---|---|---|---|---|---|---|
| 01-4167 | --- | 1.10 | 1.23 | --- | 1.01 | 1.21 |
| 01-4168 | --- | 0.41 | 0.60 | --- | 0.28 | 0.42 |
| 01-4169 | --- | 1.19 | 1.16 | --- | 1.05 | 1.12 |

### Interpretation(s)

1. The ELISA OD values were corrected for background of roughly 0.1-0.2 OD units; there was no significant difference between *Helicobacter* sp antigens in ELISA assays.
2. The challenge dose of *H. pylori* inoculum was below the colonization threshold for gnotobiotic piglets, even though all vaccinates (proteolytic digest in squalene or saline, Groups A and Groups C) seroconverted after vaccinations (Pre-challenge sera) and ELISA titers had increased slightly by termination.
3. A "priming" effect of vaccination may be evident if the responses to the vaccine digests in either the squalene adjuvant or in saline (Groups A and C) is compared to the lack of response, even after subinfectious challenge, in the challenge control group (Group B).

**Table 8. ELISA (IgG) serum antibody responses to lysates of Helicobacter species in gnotobiotic piglets orally infected with H. cerdo and terminated at 34 days of age.**

| Group & Piglet | *Helicobacter pylori* antigen: | | | *Helicobacter cerdo* antigen: | | |
|---|---|---|---|---|---|---|
| | Pre-vaccination | Pre-challenge | Terminal | Pre-vaccination | Pre-challenge | Terminal |
| 02-2662 | --- | --- | --- | --- | --- | --- |
| 02-2663 | --- | --- | --- | --- | --- | --- |
| 02-2664 | --- | --- | 0.23 | --- | --- | 0.25 |

### Interpretation(s)

1. The ELISA OD values were corrected for background of roughly 0.1-0.2 OD units; there was no significant difference between *Helicobacter* sp antigens in ELISA assays.
2. Piglet 02-2663 was lightly colonized; organisms were only recovered in re-streaks; the other two piglets had colonization levels roughly one-tenth that (e.g. 10⁵ cfu/gram) expected for *H pylori.*

**Table 9. ELISA (IgG) serum antibody responses to lysates of Helicobacter species in gnotobiotic piglets vaccinated three times with protease digests of either H. pylori or H. cerdo, challenged with H. cerdo after vaccinations and terminated at 35 days of age.**

| Group & Piglet | *Helicobacter pylori* antigen: | | | *Helicobacter cerdo* antigen: | | |
|---|---|---|---|---|---|---|
| | Pre-vaccination | Pre-challenge | Terminal | Pre-vaccination | Pre-challenge | Terminal |
| Group A: Vaccinated with *Pylori* digest and infected with *H cerdo* | | | | | | |
| 02-3481 | --- | 1.23 | 1.29 | --- | 1.20 | 1.18 |
| 02-3482 | --- | 1.11 | 1.32 | --- | 1.13 | 1.20 |

| Group B: Vaccinated with *H cerdo* digest and infected with *H cerdo* | | | | | | |
|---|---|---|---|---|---|---|
| 02-3484 | --- | --- | 0.93 | --- | 1.08 | 1.83 |
| 02-3485 | --- | 1.15 | 1.84 | --- | 0.65 | 1.44 |

| Group C: Unvaccinated and infected with *H cerdo* | | | | | | |
|---|---|---|---|---|---|---|
| 02-3483 | --- | --- | 0.09 | --- | --- | 0.11 |
| 02-3486 | --- | --- | --- | --- | --- | --- |

### Interpretation(s)

1. The ELISA OD values were corrected for background of roughly 0.1-0.2 OD units; there was no significant difference between *Helicobacter* sp antigens in ELISA assays.
2. Both digests stimulated both homologous and heterologous antibody production to specific *Helicobacter* sp antigens; there was no obvious difference in titers between homologous (same antigen for vaccination and antibody combination) and heterologous antigen (different antigen and antibody combination) ELISA systems.
3. The modest response to antigen in the challenge control piglets (Group C) is likely attributable to the fact that the challenge infection was for only 18 days (after vaccinations).

### Example 4

### Efficacy of Various Adjuvants

A number of experiments were conducted to test the efficacy of various adjuvants with the vaccine compositions, including incomplete Freund's adjuvant (ICFA) (Difco), TRIGEN (Newport Laboratories, Worthington, MN), IM-CREST 21 (Newport Laboratories, Worthington, MN) and RESPISURE (Pfizer Animal Health). Pigs administered the vaccine adjuvanted with TRIGEN showed a severe granulomatous reaction at injection sites but showed positive responses in 24-hour skin tests. Seroconversion tests on pigs administered the TRIGEN-containing vaccine showed promise. Two out of three of the pigs administered the vaccine adjuvanted with IM-CREST 21 died 48 hours after the first injection, likely due to LPS included in the adjuvant.

Parenteral vaccination using ICFA and RESPISURE prevented bacterial colonization and gastritis. However, parenteral vaccinations of actively infected piglets was not effective and may increase the histologic severity of gastritis. Therefore, antibiotic therapy could be administered prior to immunization of actively infected animals.

Immunogens in Squalene, RESPISURE and ICFA stimulated IgG isotype specific antibody responses prior to challenge. Immunogens in saline also stimulated antibody production but OD values were less than those given immunogen in adjuvants. Challenge infection with Hp/HC increased OD values in terminal sera.

Further results are shown in Tables 10-16.

**Table 10. A summary of histopathologic observations in gnotobiotic piglets vaccinated with protease digests in incomplete Freund's adjuvant, infected with H. cerdo and terminated at 35 days.**

| | | | | | | | | | |
|---|---|---|---|---|---|---|---|---|---|
| Group and Piglet ID No. | Anatomical Region of the Stomach | | | | | Nonglandular^{a} | | Gastric Lymph Nodes | Orgs present H&E |
| | | | | | | Cardia | Other | | |
| | Card | Fund | Antrum | Pylorus | | Ero Ulc | | | |

| Vaccinated with *H. pylori* digest and infected with *H. cerdo* | | | | | | | | | |
|---|---|---|---|---|---|---|---|---|---|
| 02-3481 | 2^{b} | 0 | 1 | 0 | + | - | - | reactive | - |
| 02-3482 | 2 | 1 | 2 | 0 | + | - | - | reactive | - |

| Vaccinated with *H. cerdo* digest and infected with *H. cerdo* | | | | | | | | | |
|---|---|---|---|---|---|---|---|---|---|
| 02-3484 | 1 | 0 | 1 | 0 | + | + | - | reactive | - |
| 02-3485 | 3 | 0 | 2 | 0 | not available | | - | reactive | - |

| Unvaccinated and infected with *H. cerdo* | | | | | | | | | |
|---|---|---|---|---|---|---|---|---|---|
| 02-3483 | 2 | 0 | 4 | 1 | + | + | duoden | reactive | +^{c} |
| | | | | | | | micro-ulcer | | |
| 02-3486 | 3 | 0 | 3 | 0 | + | + | - | reactive | - |

| | | | | | | | | | |
|---|---|---|---|---|---|---|---|---|---|
| ^{a} Erosions (epithelial loss restricted to epithelium superficial to basement membrane) noted in the nonglandular cardia of the stomach. Ulcerative lesions of the nonglandular cardia penetrate the basement membrane, extend and into the muscularis. The ulcer bed consists of immature granulation tissue. ^{b} Multifocal and follicular lymphocytic infiltrates into the gastric mucosa subjectively scored as 0 = no change from normal (no inflammation); 1 = minimal change from normal; 2 = moderate change from normal; 3 = severe change from normal and; 4 very severe change from normal. ^{c} Organisms detected in the hematoxylin and eosin-stained section of the antrum adjacent to gastric follicular gastritis (Warthin Starry stained sections are pending). ^{d} A micro-ulcer was detected in the duodenal mucosa of a section of duodenum present in this block. Tissues of the rest of the gastrointestinal tract were saved in formalin and will be examined. | | | | | | | | | |

**Table 11. A summary of microbial culture and reisolation results in gnotobiotic piglets vaccinated with protease digests in incomplete Freund's adjuvant, infected with H. cerdo and terminated at 35 days.**

| Group and Piglet ID | *H. cerdo* at termination (PID 35) | | | | Culture results in rest of gi track^{a,b} | | | | | |
|---|---|---|---|---|---|---|---|---|---|---|
| | No.cfu/gm (x10⁶) | Urease | Cata | Eso | Duo | Jej | Ileum Sp | Col dis | Col ter | Col |
| Vaccinated with *H. pylori* digest and infected with *H. cerdo* | | | | | | | | | | |
| 02-3481 | 5.58 | + | + | - | - | - | - | - | - | - |
| 02-3482 | 2.17 | + | + | - | - | - | - | - | - | - |

| Vaccinated with *H. cerdo* digest and infected with *H. cerdo* | | | | | | | | | | |
|---|---|---|---|---|---|---|---|---|---|---|
| 02-3484 | - | - | - | - | - | - | - | - | - | - |
| 02-3485 | 0.06 | + | + | - | - | - | - | - | - | - |

| Unvaccinated and infected with *H. cerdo* | | | | | | | | | | |
|---|---|---|---|---|---|---|---|---|---|---|
| 02-3483 | 6.40 | + | + | - | + | + | + | - | - | - |
| 02-3486 | 33.20 | + | + | - | + | - | - | - | - | - |

| | | | | | | | | | | |
|---|---|---|---|---|---|---|---|---|---|---|
| ^{a} Abbrevations used: gi = gastrointestinal tract, Eso = esophagus, Duo = Duodenum, Jej = Jejunum, Sp Col spiral Colon, dis Col = distal Colon, ter Col = terminal Colon. ^{b} reported as nd = not done, + = organisms present, - = organisms not present, (-): no growth, even upon restreaks of the plates | | | | | | | | | | |

**Table 12. A summary of gross observations in gnotobiotic piglets vaccinated^{a} with H. cerdo (Hc) proteolytic digest emulsified in ICFA and challenged with Hc 5 days after the last vaccination.**

| Group & Piglet No. | Wt. (Gms) | Gender (M/F) | Excess Mucus | Lymphoid Follicles | Submucosal Edema | Skin Test^{b} 48 hr | Ulcers and/or Erosions |
|---|---|---|---|---|---|---|---|
| Uninfected (contact infected) Controls | | | | | | | |
| 03-1100 | 1840 | F | 1^{c} | 1 | 1 | -^{d} | potential fundic mucosal ulcer, |
| GEU | | | | | | | |
| 03-1097 | 2100 | F | 1 | 2 | 1 | - | GEU (1x1 cm) |

| Vaccinated 3X with Hc and then challenged with Hc | | | | | | | |
|---|---|---|---|---|---|---|---|
| 03-1091 | 2050 | M | 1 | 1 | 1 | + | - |
| 03-1092 | 2590 | F | 1 | 2 | 1 | +/- | GEU and general congestion |
| 03-1093 | 2400 | F | 1 | 2 | 1 | - | small erosion? |
| 03-1094 | 1690 | M | 2 | 2 | 1 | +/- | - |
| 03-1095 | 2380 | M | 1 | 1 | 0 | - | - |
| 03-1096 | 2080 | M | 1 | 2 | 1 | +/- | - |

| | | | | | | | |
|---|---|---|---|---|---|---|---|
| a Immunized at 7, 10 and 17 days of age with proteolytic Hc digest in incomplete Freunds adjuvant. b Skin test antigen consisted of *H. cerdo* preparation, (10.0 ug, clarified sonicate in 0.1 ml PBS). c Visually scored as 0 = no change from normal; 1 = minimal change; 2 = moderate change; and 3 = severe change d Skin test responses scored as negative (-), positive (+) or +/- (reddening in the subcutis without obvious swelling. | | | | | | | |

**Table 13. A summary of histopathologic changes in gnotobiotic piglets vaccinated^{a} with H. cerdo (Hc) proteolytic digest emulsified in ICFA and challenged with Hc 5 days after the last vaccination.**

| Group & ID number | Anatomical Region of the Stomach | | | | | | |
|---|---|---|---|---|---|---|---|
| | Cardia | Fundus | Antrum | Pylorus | Duodeum | Gastric Lymph nodes | Skin test (24 hr) |
| Infected (challenge) Controls | | | | | | | |
| 03-1100 | 2 deep ulcer | 1 | 2 | 0 | - | reactive | 2+ |
| 03-1097 | 2 deep ulcer | 1 | 2 | 1 | - | reactive | 2+ |

| Vaccinated 3X with Hc and then challenged with He | | | | | | | |
|---|---|---|---|---|---|---|---|
| 03-1091 | 2 (-) | 1 | 1 | 0 | - | reactive | 4+ |
| 03-1092 | 1 ulcer | 0 | 2 | 0 | - | reactive | 4+ |
| 03-1093 | 2 erosion | 1 | 2 | 0 | - | reactive | 4+ |
| 03-1094 | 0 (-) | 0 | 0 | 0 | - | reactive | 4+ PMNs/hem |
| 03-1095 | 1 erosion | 1 | 0 | 0 | - | reactive | 4+ PMNs |
| 03-1096 | 1 erosion | 0 | 1 | 0 | - | reactive | 1+ |

| | | | | | | | |
|---|---|---|---|---|---|---|---|
| a H/E = hematoxylin and eosin stain; W/S = Warthin-Starry stain b Subjectively scored as 0 = no change from normal (no inflammation); 1 = minimal change from normal; 2 = moderate change from normal; and 3 = severe change from normal. c Scored as (+) for small curved extracellular microorganisms present on the gastric luminal surface of the sections or (-): no microbes seen. d GEU: gastroesophageal ulceration in the nonglandular cardia and adjacent glandular mucosa of the lesser curvature of the stomach. | | | | | | | |

**Table 14. A summary of microbiologic findings in gnotobiotic piglets vaccinated^{a} with X. cerdo (Hc) proteolytic digest emulsified in ICFA and challenged with Hc 5 days after the last vaccination.**

| Group | *Helicobacter cerdo* at termination (PID 35) | | | Other Microbial |
|---|---|---|---|---|
| and Piglet No. | cfu/gm (x10⁶) | Urease | Catalase | Contaminants |
| Infected (challenge) Controls | | | | |
| 03-1100 | 0.21 | + | + | none |
| 03-1097 | 6.61 | + | + | none |

| Vaccinated 3X with Hc and then challenged with Hc | | | | |
|---|---|---|---|---|
| 03-1091 | 0.16 | + | + | none |
| 03-1092 | 0.07 | + | + | none |
| 03-1093 | 0.93 | + | + | none |
| 03-1094 | - | - | - | none |
| 03-1095 | - | - | - | none |
| 03-1096 | 0.003 | + | + | none |

**Table 15. ELISA (IgG) serum antibody responses to lysates of Helicobacter species in gnotobiotic piglets vaccinated three times with H. pylori proteolytic digest in either RespisureR or incomplete Freund's adjuvant (ICFA), orally infected with H. pylori and terminated at 35 days of age.**

| Group & Piglet | *Helicobacter pvlori* antigen: | | | *Helicobacter cerdo* antigen: | | |
|---|---|---|---|---|---|---|
| | Pre-vaccination | Pre-challenge | Terminal | Pre-vaccination | Pre-challenge | Terminal |
| Group A: Vaccinated three times with protease digest emuslified in Respisure and challenged with *H* *pylori* | | | | | | |
| 02-2021 | --- | 1.13 | 1.45 | --- | 0.98 | 1.34 |
| 02-2022 | --- | 1.47 | 1.30 | --- | 1.06 | 1.18 |
| 02-2023 | --- | 1.14 | 1.40 | --- | 1.23 | 1.47 |

| Group B: Vaccinated three times with protease digest in incomplete Freunds adjuvant and challenged with *H pylori* | | | | | | |
|---|---|---|---|---|---|---|
| 02-2024 | --- | 1.26 | 1.89 | --- | 0.80 | 1.39 |
| 02-2025 | --- | 1.41 | 1.92 | --- | 1.35 | 1.63 |
| 02-2026 | --- | 1.34 | 1.64 | --- | 1.42 | 1.44 |

| Group C: Challenged with *H pylori* | | | | | | |
|---|---|---|---|---|---|---|
| 02-2027 | --- | --- | --- | --- | --- | --- |
| 02-2028 | --- | --- | 0.27 | --- | --- | 0.12 |

### Interpretation(s)

1. The ELISA OD values were corrected for background of roughly 0.1-0.2 OD units; there was no significant difference between *Helicobacter* sp antigens in ELISA assays.
2. Both the RespisureR and ICFA adjuvants stimulated significant ELISA titers to *Helicobacter* sp antigens prior to challenge with *H. pylori.*
3. One of two unvaccinated control pigs challenged with *H. pylori* seroconverted; this "slow" serologic response has been seen in previous challenge experiments in that it takes several weeks to detect IgG antibodies and the challenge to termination interval was only 15 days.

**Table 16. ELISA (IgG) serum antibody responses to lysates of Helicobacter species in gnotobiotic piglets vaccinated three times with H. pylori proteolytic digest, orally infected with a suboptimal amount of H. pylori and terminated at 24 days of age.**

| Group & Piglet | *Helicobacter pylori* antigen: | | | *Helicobacter cerdo* antigen: | | |
|---|---|---|---|---|---|---|
| | Pre-vaccination | Pre-challenge | Terminal | Pre-vaccination | Pre-challenge | Terminal |
| Group A: Vaccinated three times with saline alone and challenged with *H pylori* | | | | | | |
| 02-741 | --- | --- | --- | --- | --- | --- |
| 02-742 | --- | --- | --- | --- | --- | --- |
| 02-743 | --- | --- | --- | --- | --- | --- |
| Group B: Vaccinated three times with protease digest in saline and challenged with *H pylori* | | | | | | |
| 02-744 | --- | 0.75 | 0.75 | --- | 0.67 | 0.74 |
| 02-745 | --- | 1.11 | 0.78 | --- | 1.08 | 0.78 |
| 02-746 | --- | 0.73 | 1.05 | --- | 0.73 | 0.98 |
| Group C: Vaccinated three times with protease digest in TRIGEN adjuvant (Newport Laboratories) and challenged with *H pylori* | | | | | | |
| 02-750 | (ELISAs in progress) | | | | | |
| 02-751 | | | | | | |
| 02-752 | | | | | | |
| Group D: Vaccinated with protease digest in IM-CREST 21 adjuvant (Newport Laboratories) | | | | | | |
| 02-747 | --- | died 48 hrs after the first vaccination | | | | |
| 02-748 | --- | --- | --- | --- | --- | 0.25 |
| 02-749 | --- | died 48 hrs after the first vaccination | | | | |

### Interpretation(s)

1. The ELISA OD values were corrected for background of roughly 0.1-0.2 OD units; there is no significant difference between *Helicobacter* sp antigens in ELISA assays.

### Example 5

### Characterization of H. cerdo and H. pylori

In order to demonstrate that *H. cerdo* was in fact a distinct organism from *H. pylori,* SDS-PAGE gels were run under reducing conditions to examine the protein profiles of the two organisms. The stacking gel for separation consisted of 3.9% acrylamide; the separating gel contained 12% acrylamide. Each was made using standard procedures as outlined in *Current Protocols in Molecular Biology, supplement 47, section 10.2A.6.* In some instances, native PAGE gels were used that were purchased from BioRad Corporation. The gel loading buffer consisted of Tris-Cl (50 mM), pH 6.8, 2% SDS (electrophoresis grade), 0.1% bromophenol blue and 10% glycerol. Samples were run in a Tris-glycine buffer containing 25 mM Tris, 250 mM glycine (electrophoresis grade, pH 8.3) and 0.1% SDS.

The samples consisted of intact and digested *H. pylori* (Hp) and *H. cerdo* (Hc). The proteolytic digests were done as described above. One µl of each sample (2.4-3.0 µg) was diluted in distilled water to a final volume of 15 µl and diluted 1:2 with loading buffer. Samples were boiled for 3 minutes, and 20 µl of each sample loaded onto the gel. Samples (along with a standard) were then electrophoresed at 100 V for 60-75 minutes or until the dye fronts had just exited the gels. Gels were then stained with Coomassie Blue or silver stains to develop the separated bands and then photographed. Following clearing in dilute acetic acid solution overnight, gels were dehydrated and then photographed.

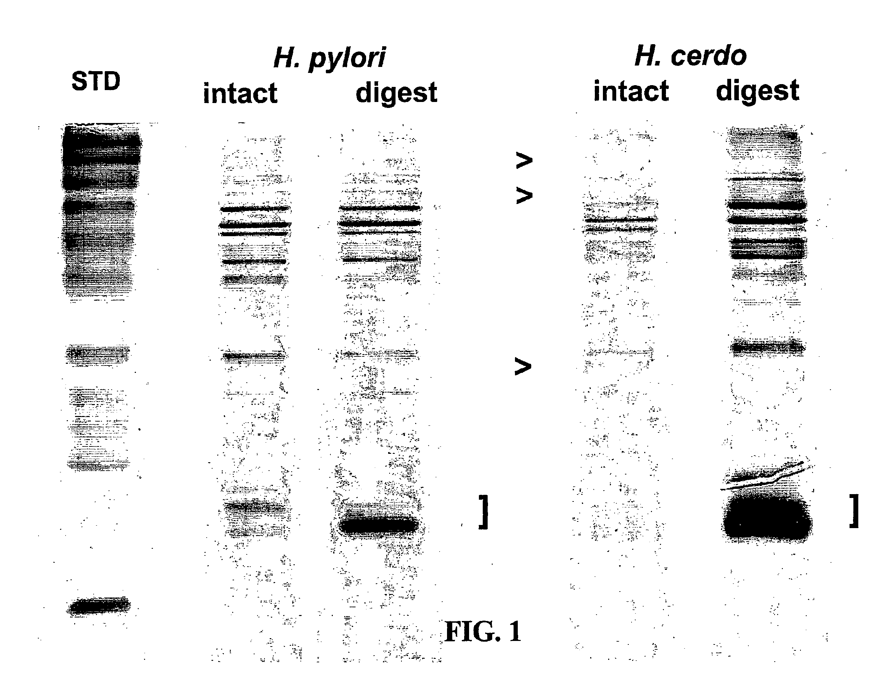

As shown in Figure 1, the SDS-PAGE profiles of both intact and digested *H*. *pylori* and *H. cerdo* were different. The ">" in the figure illustrates bands present in Hp and absent from Hc. The "]" indicates low molecular weight protease digest products.

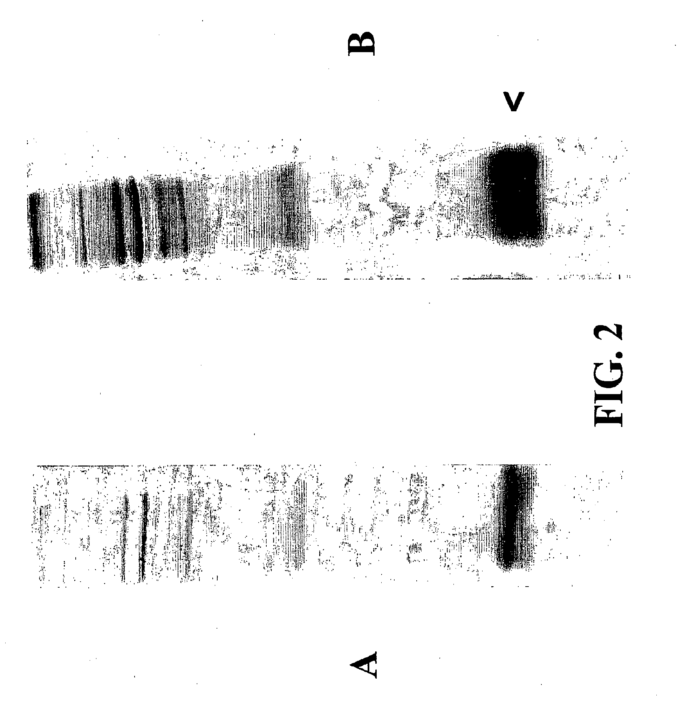

SDS-PAGE gels of intact and digested *H. cerdo* were also run and compared. As can be seen in Figure 2A (intact) and 2B (digested), an increased amount of low molecular weight material was present in the proteolytic digestion product (indicated by "<" in Figure 2B.

Western blot analysis of intact *H. cerdo* and digested *H. cerdo* was also performed. Samples were separated on PAGE gels (reducing and native gels, as described above) and were transferred to nitrocellulose membranes by standard electrophoretic methodology using a BioRad apparatus. Nitrocellulose membranes were incubated overnight (4°C) in phosphate buffered saline containing 10% nonfat dry milk containing TWEEN 20 (PBS-NFM) to block reactive sites on the membranes. After washing, a 1:250 dilution of porcine serum (diluted in PBS-NFM) was made and incubated for 2 hr at 22°C. After washing 3 times (5 minutes each) in PBS-NFM, membranes were incubated with goat anti-porcine IgG, for one hr at 22°C. The membranes were washed again as above and developed with warmed (37°C) TMB membrane horse radish peroxidase substrate for several minutes. The reaction was stopped by the addition of excess distilled water. Membranes were then dried and photographed.

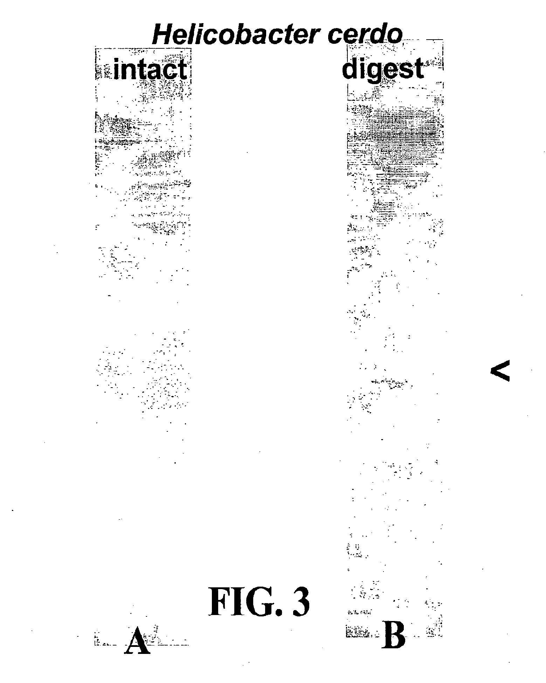

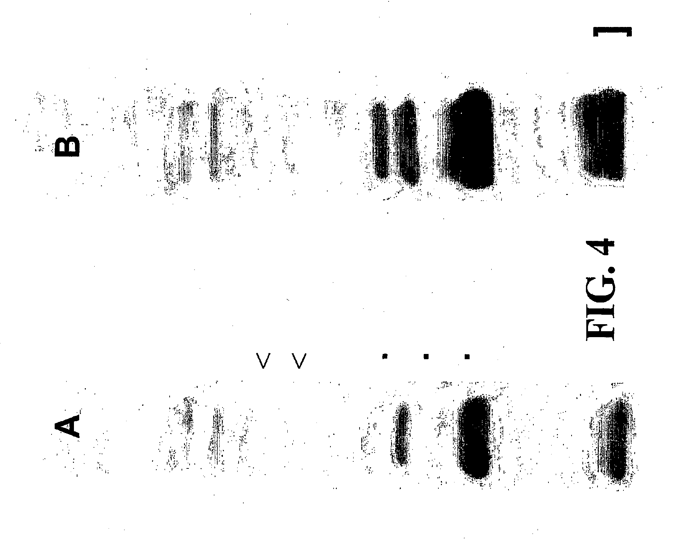

As seen in Figures 3A and 3B, the low molecular weight material present in Figure 2B enters the native gel and is immunoreactive with test sera from pigs. As shown in Figures 4A and 4B, Western blot analysis of the antibody reactivity profile against intact *H. cerdo* (4A) and an *H. cerdo* digest (4B) showed an increased amount of low molecular weight material in the digest (indicated by ]). Increased staining intensity was also seen (•), as well as additional immunoreactive bands (<). As is apparent, the *H. cerdo* lysate contains immunoreactive material that cross-reacts with the intact organism, indicating that this is likely the basis for protection. Moreover, prevaccination sera were negative and post-vaccination/post-challenge sera were strongly positive.

Thus, compositions for use in methods for treating, preventing and diagnosing *Helicobacter* infection are described.

## Claims

1. A composition comprising a pharmaceutically acceptable vehicle and at least one *Helicobacter cerdo* immunogen; wherein said *Helicobacter cerdo* is a porcine *Helicobacter* gastric isolate which has the following characteristics: gram negative, short curved rods, microaerophilic growth pattern, urease enzyme activity, catalase enzyme activity, possession of one or more immunogenic polypeptides selected from the group consisting of HpaA, FlaA, FlaB, urease, UreA, UreB, hsp60, cytotoxin, cagA, and VacA, and a distinct SDS-PAGE profile from *Helicobacter pylori* under reducing conditions; and wherein the at least *one H. cerdo* immunogen is provided in an *H. cerdo* lysate, wherein the *H. cerdo* lysate is produced by proteolytic digestion of *H. cerdo* bacteria.

2. The composition of claim 1 wherein the enzyme used in the proteolytic digestion is pepsin.

3. The composition of claim 1 or 2, further comprising an adjuvant.

4. A method of producing a composition comprising:
(a) providing at least one *Helicobacter cerdo* immunogen; and
(b) combining said *H. cerdo* immunogen with a pharmaceutically acceptable vehicle;
wherein said *Helicobacter cerdo* is a porcine *Helicobacter* gastric isolate which has the following characteristics: gram negative, short curved rods, microaerophilic growth pattern, urease enzyme activity, catalase enzyme activity, possession of one or more immunogenic polypeptides selected from the group consisting of HpaA, FlaA, FlaB, urease, UreA, UreB, hsp60, cytotoxin, cagA, and VacA, and a distinct SDS-PAGE profile from *Helicobacter pylori* under reducing condition; and wherein said at least one *H. cerdo* immunogen is provided in *an H. cerdo* lysate, wherein the *H. cerdo* lysate is produced by proteolytic digestion *of H. cerdo* bacteria.

5. The method of claim 4 wherein the enzyme used in the proteolytic digestion is pepsin.

6. The method of claim 4 or 5, further comprising providing an adjuvant.

7. A method of detecting *Helicobacter cerdo* infection in a vertebrate subject comprising:
(a) providing a biological sample from the subject; and
(b) reacting said biological sample with at least one *H. cerdo* immunogen, under conditions which allow *Helicobacter* antibodies, when present in the biological sample, to bind with said immunogen(s),
thereby detecting the presence or absence of *Helicobacter cerdo* infection in the subject;
wherein said *Helicobacter cerdo* is a porcine *Helicobacter* gastric isolate which has the following characteristics: gram negative, short curved rods, microaerophilic growth pattern, urease enzyme activity, catalase enzyme activity, possession of one or more immunogenic polypeptides selected from the group consisting of HpaA, FlaA, FlaB, urease, UreA, UreB, hsp60, cytotoxin, cagA, and VacA, and a distinct SDS-PAGE profile from *Helicobacter pylori* under reducing condition; and, wherein said at least one immunogen is provided in an *H. cerdo* lysate, wherein the *H. cerdo* lysate is produced by proteolytic digestion *of H. cerdo* bacteria.

8. The method of claim 7 further comprising:
(c) removing unbound antibodies;
(d) providing one or more moieties capable of associating with said bound antibodies; and
(e) detecting the presence or absence of said one or more moieties, thereby detecting the presence or absence *of H. cerdo* infection.

9. The method of claim 8 wherein a fluorescer or an enzyme label is used to detect the presence or absence of *Helicobacter cerdo* infection in the subject.

10. The method of any one of claims 7 to 9, wherein said biological sample is a porcine serum sample.

11. The method of any one of claims 7 to 10 wherein the enzyme used in the proteolytic digestion is pepsin.

12. An antibody specific for a *Helicobacter cerdo* immunogen; wherein said *Helicobacter cerdo* is a porcine *Helicobacter* gastric isolate which has the following characteristics: gram negative, short curved rods, microaerophilic growth pattern, urease enzyme activity, catalase enzyme activity, possession of one or more immunogenic polypeptides selected from the group consisting of HpaA, FlaA, FlaB, urease, UreA, UreB, hsp60, cytotoxin, cagA, and VacA, and a distinct SDS-PAGE profile from *Helicobacter pylori* under reducing condition; and wherein the *H. cerdo* immunogen is provided in an *H. cerdo* lysate, wherein the *H. cerdo* lysate is produced by proteolytic digestion *of H. cerdo* bacteria.

13. The antibody of claim 12 wherein the enzyme used in the proteolytic digestion is pepsin.

14. The antibody of claim 12, wherein the antibody is a polyclonal antibody.

15. The antibody of claim 12, wherein the antibody is a monoclonal antibody.

16. A *Helicobacter cerdo* lysate comprising at least one *H. cerdo* immunogen; wherein said *Helicobacter cerdo* is a porcine *Helicobacter* gastric isolate which has the following characteristics: gram negative, short curved rods, microaerophilic growth pattern, urease enzyme activity, catalase enzyme activity, possession of one or more immunogenic polypeptides selected from the group consisting of HpaA, FlaA, FlaB, urease, UreA, UreB, hsp60, cytotoxin, cagA, and VacA, and a distinct SDS-PAGE profile from *Helicobacter pylori* under reducing condition; wherein the *H. cerdo* lysate is produced by proteolytic digestion of *H. cerdo* bacteria.

17. The *H. cerdo* lysate of claim 16 wherein the enzyme used in the proteolytic digestion is pepsin.

18. Use of a composition according to any of claims 1 to 3 or an *H. cerdo* lysate according to claim 16 or 17 for the manufacture of a medicament for the treatment or prevention of a *Helicobacter cerdo* infection in a vertebrate subject; wherein said *Helicobacter cerdo* is a porcine *Helicobacter* gastric isolate which has the following characteristics: gram negative, short curved rods, microaerophilic growth pattern, urease enzyme activity, catalase enzyme activity, possession of one or more immunogenic polypeptides selected from the group consisting of HpaA, FlaA, FlaB, urease, UreA, UreB, hsp60, cytotoxin, cagA, and VacA, and a distinct SDS-PAGE profile from *Helicobacter pylori* under reducing condition.

19. The use according to claim 20 wherein said composition or *H. cerdo* lysate is in a therapeutically effective amount.

20. The use according to claim 18 or 19, wherein said vertebrate subject is a porcine subject.

21. The use according to any one of claims 18 to 20, wherein said composition or *H. cerdo* lysate is for parental administration.

22. Use of an *H. cerdo* lysate according to claim 16 or 17 in an *ex vivo* method of detecting *Helicobacter cerdo* infection in a vertebrate subject; wherein said *Helicobacter cerdo* is a porcine *Helicobacter* gastric isolate which has the following characteristics: gram negative, short curved rods, microaerophilic growth pattern, urease enzyme activity, catalase enzyme activity, possession of one or more immunogenic polypeptides selected from the group consisting of HpaA, FlaA, FlaB, urease, UreA, UreB, hsp60, cytotoxin, cagA, and VacA, and a distinct SDS-PAGE profile from *Helicobacter pylori* under reducing condition.

23. The use of claim 22, wherein the vertebrate subject is a porcine subject.

24. A composition according to any of claims 1 to 3 or an *H. cerdo* lysate according to claim 16 or 17 for use in the treatment or prevention of a *Helicobacter* infection in a vertebrate subject; wherein said *Helicobacter cerdo* is a porcine *Helicobacter* gastric isolate which has the following characteristics: gram negative, short curved rods, microaerophilic growth pattern, urease enzyme activity, catalase enzyme activity, possession of one or more immunogenic polypeptides selected from the group consisting of HpaA, FlaA, FlaB, urease, UreA, UreB, hsp60, cytotoxin, cagA, and VacA, and a distinct SDS-PAGE profile from *Helicobacter pylori* under reducing condition.

25. The composition or *H. cerdo* lysate for use according to claim 24 wherein said composition or *H. cerdo* lysate is in a therapeutically effective amount.

26. The composition or *H. cerdo* lysate for use according to claim 25, wherein said vertebrate subject is a porcine subject.

27. The composition or *H. cerdo* lysate for use according to any one of claims 24 to 26, wherein said composition or *H. cerdo* lysate is administered parenterally.

28. A composition comprising a *Helicobacter cerdo* lysate according to claim 16 or 17 for use in a method for producing an immunological response against *Helicobacter* in a porcine.

## Patentansprüche

1. Zusammensetzung umfassend einen pharmazeutisch verträglichen Träger und mindestens ein *Helicobacter cerdo*-Immunogen; wobei der *Helicobacter cerdo* ein porciner *Helicobacter* eines Magenisolats ist, der die folgenden Eigenschaften hat: gram-negativ, kurze gekrümmte Stäbchen, mikroaerophiles Wachstumsmuster, Urease-Enzymaktivität, Katalase-Enzymaktivität, Besitz von einem oder mehreren immunogenen Polypeptiden, die ausgewählt sind aus der Gruppe bestehend aus HpaA, FlaA, FlaB, Urease, UreA, UreB, hsp60, Zytotoxin, cagA und VacA und ein sich von *Helicobacter pylori* unterscheidendes SDS-PAGE-Profil unter reduzierenden Bedingungen; und wobei das mindestens eine *H. cerdo*-Immunogen in einem *H. cerdo-Lysat* bereitgestellt ist, wobei das *H*. *cerdo-Lysat* durch proteolytischen Verdau von *H. cerdo*-Bakterien hergestellt wird.

2. Zusammensetzung nach Anspruch 1, wobei das Enzym, das in dem proteolytischen Verdau verwendet wird, Pepsin ist.

3. Zusammensetzung nach Anspruch 1 oder 2, die zudem einen Hilfsstoff umfasst.

4. Verfahren zur Herstellung einer Zusammensetzung, umfassend:
(a) Bereitstellen von mindestens einem *Helicobacter cerdo*-Immunogen; und
(b) Kombinieren des *H. cerdo*-Immunogens mit einem pharmazeutisch verträglichen Träger;
wobei der *Helicobacter cerdo* ein porciner *Helicobacter* eines Magenisolats ist, der die folgenden Eigenschaften hat: gram-negativ, kurze gekrümmte Stäbchen, mikroaerophiles Wachstumsmuster, Urease-Enzymaktivität, Katalase-Enzymaktivität, Besitz von einem oder mehreren immunogenen Polypeptiden, die ausgewählt sind aus der Gruppe bestehend aus HpaA, FlaA, FlaB, Urease, UreA, UreB, hsp60, Zytotoxin, cagA und VacA und ein sich von *Helicobacter pylori* unterscheidendes SDS-PAGE-Profil unter reduzierenden Bedingungen;
und wobei das mindestens eine *H. cerdo*-Immunogen in einem *H. cerdo-*Lysat bereitgestellt ist, wobei das *H*. *cerdo-Lysat* durch proteolytischen Verdau von *H*. *cerdo*-Bakterien hergestellt wird.

5. Verfahren nach Anspruch 4, wobei das Enzym, das in dem proteolytischen Verdau verwendet wird, Pepsin ist.

6. Verfahren nach Anspruch 4 oder 5, das zudem einen Hilfsstoff umfasst.

7. Verfahren zum Nachweis einer *Helicobacter cerdo*-Infektion in einem Wirbeltierindividuum, umfassend:
(a) Bereitstellen einer biologischen Probe von dem Individuum; und
(b) Reagieren der biologischen Probe mit mindestens einem *H. cerdo*Immunogen,
unter Bedingungen, die es den *Helicobacter-Antikörpern* ermöglichen, wenn diese in der biologischen Probe vorhanden sind, an das/die Immunogen(e) zu binden,
um dadurch das Vorhandensein oder Nichtvorhandensein der *Helicobacter cerdo*-Infektion in dem Individuum nachzuweisen;
wobei der *Helicobacter cerdo* ein porciner *Helicobacter* eines Magenisolats ist, der die folgenden Eigenschaften hat: gram-negativ, kurze gekrümmte Stäbchen, mikroaerophiles Wachstumsmuster, Urease-Enzymaktivität, Katalase-Enzymaktivität, Besitz von einem oder mehreren immunogenen Polypeptiden, die ausgewählt sind aus der Gruppe bestehend aus HpaA, FlaA, FlaB, Urease, UreA, UreB, hsp60, Zytotoxin, cagA und VacA und ein sich von *Helicobacter pylori* unterscheidendes SDS-PAGE-Profil unter reduzierenden Bedingungen; und wobei das mindestens eine *H*. *cerdo*-Immunogen in einem *H. cerdo-*Lysat bereitgestellt ist, wobei das *H*. *cerdo-Lysat* durch proteolytischen Verdau von *H*. *cerdo*-Bakterien hergestellt wird.

8. Verfahren nach Anspruch 8, weiterhin umfassend:
(c) Entfernen von ungebundenen Antikörpern;
(d) Bereitstellen einer oder mehrerer Reste, die dazu in der Lage sind, mit den gebundenen Antikörpern zu assoziieren; und
(e) Nachweisen des Vorhandenseins oder Nichtvorhandenseins der ein oder mehreren Reste,
um dadurch das Vorhandensein oder Nichtvorhandensein der *H*. *cerdo*-Infektion nachzuweisen.

9. Verfahren nach Anspruch 8, wobei eine Fluoreszenz- oder eine Enzymmarkierung verwendet wird, um das Vorhandensein oder Nichtvorhandensein der *Helicobacter cerdo*-Infektion in dem Individuum nachzuweisen.

10. Verfahren nach einem der Ansprüche 7 bis 9, wobei die biologische Probe eine porcine Serumprobe ist.

11. Verfahren nach einem der Ansprüche 7 bis 10, wobei das Enzym, das in dem proteolytischen Verdau verwendet wird, Pepsin ist.

12. Antikörper, der spezifisch für ein *Helicobacter cerdo*-Immunogen ist; wobei der *Helicobacter cerdo* ein porciner *Helicobacter* eines Magenisolats ist, der die folgenden Eigenschaften hat: gram-negativ, kurze gekrümmte Stäbchen, mikroaerophiles Wachstumsmuster, Urease-Enzymaktivität, Katalase-Enzymaktivität, Besitz von einem oder mehreren immunogenen Polypeptiden, die ausgewählt sind aus der Gruppe bestehend aus HpaA, FlaA, FlaB, Urease, UreA, UreB, hsp60, Zytotoxin, cagA und VacA und ein sich von *Helicobacter pylori* unterscheidendes SDS-PAGE-Profil unter reduzierenden Bedingungen; und wobei das mindestens eine *H*. *cerdo*-Immunogen in einem *H. cerdo-*Lysat bereitgestellt ist, wobei das *H*. *cerdo-Lysat* durch proteolytischen Verdau von *H*. *cerdo*-Bakterien hergestellt wird.

13. Antikörper nach Anspruch 12, wobei das Enzym, das in dem proteolytischen Verdau verwendet wird, Pepsin ist.

14. Antikörper nach Anspruch 12, wobei der Antikörper ein polyclonaler Antikörper ist.

15. Antikörper nach Anspruch 12, wobei der Antikörper ein monoclonaler Antikörper ist.

16. *Helicobacter cerdo-Lysat* umfassend mindestens ein *H*. *cerdo*-Immunogen; wobei der *Helicobacter cerdo* ein porciner *Helicobacter* eines Magenisolats ist, der die folgenden Eigenschaften hat: gram-negativ, kurze gekrümmte Stäbchen, mikroaerophiles Wachstumsmuster, Urease-Enzymaktivität, Katalase-Enzymaktivität, Besitz von einem oder mehreren immunogenen Polypeptiden, die ausgewählt sind aus der Gruppe bestehend aus HpaA, FlaA, FlaB, Urease, UreA, UreB, hsp60, Zytotoxin, cagA und VacA und ein sich von *Helicobacter pylori* unterscheidendes SDS-PAGE-Profil unter reduzierenden Bedingungen; und wobei das mindestens eine *H*. *cerdo*-Immunogen in einem *H. cerdo-*Lysat bereitgestellt ist, wobei das *H*. *cerdo-Lysat* durch proteolytischen Verdau von *H*. *cerdo*-Bakterien produziert wird.

17. *H. cerdo-Lysat* nach Anspruch 16, wobei das Enzym, das in dem proteolytischen Verdau verwendet wird, Pepsin ist.

18. Verwendung einer Zusammensetzung nach einem der Ansprüche 1 bis 3 oder *H. cerdo-Lysat* nach Anspruch 16 oder 17 zur Herstellung eines Medikaments zur Behandlung oder Vorbeugung einer *Helicobacter cerdo*-Infektion in einem Wirbeltierindividuum; wobei der *Helicobacter cerdo* ein porciner *Helicobacter* eines Magenisolats ist, der die folgenden Eigenschaften hat: gram-negativ, kurze gekrümmte Stäbchen, mikroaerophiles Wachstumsmuster, Urease-Enzymaktivität, Katalase-Enzymaktivität, Besitz von einem oder mehreren immunogenen Polypeptiden, die ausgewählt sind aus der Gruppe bestehend aus HpaA, FlaA, FlaB, Urease, UreA, UreB, hsp60, Zytotoxin, cagA und VacA und ein sich von *Helicobacter pylori* unterscheidendes SDS-PAGE-Profil unter reduzierenden Bedingungen.

19. Verwendung nach Anspruch 20, wobei die Zusammensetzung oder das *H*. *cerdo-*Lysat in einer therapeutisch wirksamen Menge ist.

20. Verwendung nach Anspruch 18 oder 19, wobei das Wirbeltierindividuum ein porcines Individuum ist.

21. Verwendung nach einem der Ansprüche 18 bis 20, wobei die Zusammensetzung oder das *H*. *cerdo-*Lysat für parenterale Verabreichung ist.

22. Verwendung eines *H*. *cerdo-*Lysats nach Anspruch 16 oder 17 in einem *ex vivo-*Verfahren zum Nachweis einer *Helicobacter cerdo*-Infektion in einem Wirbeltierindividuum; wobei der *Helicobacter cerdo* ein porciner *Helicobacter* eines Magenisolats ist, der die folgenden Eigenschaften hat: gram-negativ, kurze gekrümmte Stäbchen, mikroaerophiles Wachstumsmuster, Urease-Enzymaktivität, Katalase-Enzymaktivität, Besitz von einem oder mehreren immunogenen Polypeptiden, die ausgewählt sind aus der Gruppe bestehend aus HpaA, FlaA, FlaB, Urease, UreA, UreB, hsp60, Zytotoxin, cagA und VacA und ein sich von *Helicobacter pylori* unterscheidendes SDS-PAGE-Profil unter reduzierenden Bedingungen.

23. Verwendung nach Anspruch 22, wobei das Wirbeltierindividuum ein porcines Individuum ist.

24. Zusammensetzung nach einem der Ansprüche 1 bis 3 oder ein *H. cerdo-Lysat* nach Anspruch 16 oder 17 zur Verwendung in der Behandlung oder Vorbeugung einer *Helicobacter cerdo*-Infektion in einem Wirbeltierindividuum; wobei der *Helicobacter cerdo* ein porciner *Helicobacter* eines Magenisolats ist, der die folgenden Eigenschaften hat: gram-negativ, kurze gekrümmte Stäbchen, mikroaerophiles Wachstumsmuster, Urease-Enzymaktivität, Katalase-Enzymaktivität, Besitz von einem oder mehreren immunogenen Polypeptiden, die ausgewählt sind aus der Gruppe bestehend aus HpaA, FlaA, FlaB, Urease, UreA, UreB, hsp60, Zytotoxin, cagA und VacA und ein sich von *Helicobacter pylori* unterscheidendes SDS-PAGE-Profil unter reduzierenden Bedingungen.

25. Zusammensetzung oder *H*. *cerdo-Lysat* zur Verwendung nach Anspruch 24, wobei die Zusammensetzung oder das *H. cerdo-Lysat* in einer therapeutisch wirksamen Menge ist.

26. Zusammensetzung oder *H*. *cerdo-Lysat* zur Verwendung nach Anspruch 25, wobei das Wirbeltierindividuum ein porcines Individuum ist.

27. Zusammensetzung oder *H*. *cerdo-*Lysat zur Verwendung nach einem der Ansprüche 24 bis 26, wobei die Zusammensetzung oder das *H. cerdo-*Lysat parenteral verabreicht wird.

28. Zusammensetzung umfassend ein *Helicobacter cerdo-*Lysat nach Anspruch 16 oder 17 zur Verwendung in einem Verfahren zum Auslösen einer immunologischen Antwort gegen *Helicobacter* in einem Schwein.

## Revendications

1. Composition comprenant un véhicule pharmaceutiquement acceptable et au moins un agent immunogène *Helicobacter cerdo ;* dans laquelle ledit *Helicobacter cerdo* est un isolat gastrique *d'Helicobacter* porcin qui a les caractéristiques suivantes : gram négatif, bâtons courts recourbés, schéma de croissance micro-aérophile, activité de l'enzyme uréase, activité de l'enzyme catalase, possession d'un ou plusieurs polypeptides immunogènes choisis dans le groupe constitué de HpaA, FlaA, FlaB, uréase, UreA, UreB, hsp60, cytotoxine, cagA et VacA, et un profil SDS-PAGE distinct de celui d'Helicobacter pylori dans des conditions réductrices ; et dans laquelle l'au moins un agent immunogène *H. cerdo* est fourni dans un lysat de *H. cerdo,* dans laquelle le lysat de *H. cerdo* est produit par digestion protéolytique de la bactérie *H. cerdo.*

2. Composition selon la revendication 1, dans laquelle l'enzyme utilisée dans la digestion protéolytique est la pepsine.

3. Composition selon la revendication 1 ou 2, comprenant en outre un adjuvant.

4. Procédé de production d'une composition comprenant :
(a) la fourniture d'au moins un agent immunogène *Helicobacter cerdo ;* et
(b) la combinaison dudit agent immunogène *H. cerdo* avec un véhicule pharmaceutiquement acceptable ;
dans lequel ledit *Helicobacter cerdo* est un isolat gastrique *d'Helicobacter* porcin qui a les caractéristiques suivantes : gram négatif, bâtons courts recourbés, schéma de croissance micro-aérophile, activité de l'enzyme uréase, activité de l'enzyme catalase, possession d'un ou plusieurs polypeptides immunogènes choisis dans le groupe constitué de HpaA, FlaA, FlaB, uréase, UreA, UreB, hsp60, cytotoxine, cagA et VacA, et un profil SDS-PAGE distinct de celui d'Helicobacter pylori dans des conditions réductrices ; et dans laquelle ledit au moins un agent immunogène *H*. *cerdo* est fourni dans un lysat de *H. cerdo,* dans laquelle le lysat de *H. cerdo* est produit par digestion protéolytique de la bactérie *H. cerdo.*

5. Procédé selon la revendication 4, dans lequel l'enzyme utilisée dans la digestion protéolytique est la pepsine.

6. Procédé selon la revendication 4 ou 5, comprenant en outre la fourniture d'un adjuvant.

7. Procédé de détection d'une infection à *Helicobacter cerdo* chez un sujet vertébré comprenant :
(a) la fourniture d'un échantillon biologique du sujet ; et
(b) la réaction dudit échantillon biologique avec au moins un agent immunogène *H*. *cerdo,* dans des conditions qui permettent aux anticorps *d'Helicobacter,* lorsqu'ils sont présents dans l'échantillon biologique, de se fixer audit/auxdits agent(s) immunogène(s),
en détectant ainsi la présence ou l'absence d'une infection à *Helicobacter cerdo* chez le sujet ;
dans lequel ledit *Helicobacter cerdo* est un isolat gastrique *d'Helicobacter* porcin qui a les caractéristiques suivantes : gram négatif, bâtons courts recourbés, schéma de croissance micro-aérophile, activité de l'enzyme uréase, activité de l'enzyme catalase, possession d'un ou plusieurs polypeptides immunogènes choisis dans le groupe constitué de HpaA, FlaA, FlaB, uréase, UreA, UreB, hsp60, cytotoxine, cagA et VacA, et un profil SDS-PAGE distinct de celui *d'Helicobacter pylori* dans des conditions réductrices ; et dans lequel ledit au moins un agent immunogène *H. cerdo* est fourni dans un lysat de *H. cerdo,* dans lequel le lysat de *H. cerdo* est produit par digestion protéolytique de la bactérie *H. cercle.*

8. Procédé selon la revendication 7, comprenant en outre :
(c) le retrait des anticorps non fixés ;
(d) la fourniture d'une ou plusieurs fractions capables de s'associer avec lesdits anticorps fixés ; et
(e) la détection de la présence ou de l'absence desdites une ou plusieurs fractions, en détectant ainsi la présence ou l'absence d'une infection à *H. cerdo.*

9. Procédé selon la revendication 8, dans lequel un agent fluorescent ou un marqueur enzymatique est utilisé pour détecter la présence ou l'absence d'une infection à *Helicobacter cerdo* chez le sujet.

10. Procédé selon l'une quelconque des revendications 7 à 9, dans lequel ledit échantillon biologique est un échantillon de sérum porcin.

11. Procédé selon l'une quelconque des revendications 7 à 10, dans lequel l'enzyme utilisée dans la digestion protéolytique est la pepsine.

12. Anticorps spécifique pour un agent immunogène *Helicobacter cerdo ;* dans lequel ledit *Helicobacter cerdo* est un isolat gastrique *d'Helicobacter* porcin qui a les caractéristiques suivantes : gram négatif, bâtons courts recourbés, schéma de croissance micro-aérophile, activité de l'enzyme uréase, activité de l'enzyme catalase, possession d'un ou plusieurs polypeptides immunogènes choisis dans le groupe constitué de HpaA, FlaA, FlaB, uréase, UreA, UreB, hsp60, cytotoxine, cagA et VacA, et un profil SDS-PAGE distinct de celui *d'Helicobacter pylori* dans des conditions réductrices ; et dans lequel l'agent immunogène *H. cerdo* est fourni dans un lysat de *H. cerdo,* dans lequel le lysat de *H. cerdo* est produit par digestion protéolytique de la bactérie *H. cerdo.*

13. Anticorps selon la revendication 12, dans lequel l'enzyme utilisée dans la digestion protéolytique est la pepsine.

14. Anticorps selon la revendication 12, dans lequel l'anticorps est un anticorps polyclonal.

15. Anticorps selon la revendication 12, dans lequel l'anticorps est un anticorps monoclonal.

16. Lysat *d'Helicobacter cerdo* comprenant au moins un agent immunogène *H. cerdo ;* dans lequel ledit *Helicobacter cerdo* est un isolat gastrique *d'Helicobacter* porcin qui a les caractéristiques suivantes : gram négatif, bâtons courts recourbés, schéma de croissance micro-aérophile, activité de l'enzyme uréase, activité de l'enzyme catalase, possession d'un ou plusieurs polypeptides immunogènes choisis dans le groupe constitué de HpaA, FlaA, FlaB, uréase, UreA, UreB, hsp60, cytotoxine, cagA et VacA, et un profil SDS-PAGE distinct de celui *d'Helicobacter pylori* dans des conditions réductrices ; dans lequel le lysat de *H. cerdo* est produit par digestion protéolytique de la bactérie *H. cerdo.*

17. Lysat de *H. cerdo* selon la revendication 16, dans lequel l'enzyme utilisée dans la digestion protéolytique est la pepsine.

18. Utilisation d'une composition selon l'une quelconque des revendications 1 à 3 ou d'un lysat de *H. cerdo* selon la revendication 16 ou 17 pour la production d'un médicament pour le traitement ou la prévention d'une infection à *Helicobacter cerdo* chez un sujet vertébré ; dans laquelle ledit *Helicobacter cerdo* est un isolat gastrique *d'Helicobacter* porcin qui a les caractéristiques suivantes : gram négatif, bâtons courts recourbés, schéma de croissance micro-aérophile, activité de l'enzyme uréase, activité de l'enzyme catalase, possession d'un ou plusieurs polypeptides immunogènes choisis dans le groupe constitué de HpaA, FlaA, FlaB, uréase, UreA, UreB, hsp60, cytotoxine, cagA et VacA, et un profil SDS-PAGE distinct de celui *d'Helicobacter pylori* dans des conditions réductrices.

19. Utilisation selon la revendication 20, dans laquelle ladite composition ou ledit lysat de *H. cerdo* est en une quantité thérapeutiquement efficace.

20. Utilisation selon la revendication 18 ou 19, dans laquelle ledit sujet vertébré est un sujet porcin.

21. Utilisation selon l'une quelconque des revendications 18 à 20, dans laquelle la composition ou le lysat de *H. cerdo* est destiné(e) à l'administration parentérale.

22. Utilisation d'un lysat de *H. cerdo* selon la revendication 16 ou 17, dans un procédé *ex vivo* de détection d'une infection à *Helicobacter cerdo* chez un sujet vertébré ; dans laquelle ledit *Helicobacter cerdo* est un isolat gastrique d'*Helicobacter* porcin qui a les caractéristiques suivantes : gram négatif, bâtons courts recourbés, schéma de croissance micro-aérophile, activité de l'enzyme uréase, activité de l'enzyme catalase, possession d'un ou plusieurs polypeptides immunogènes choisis dans le groupe constitué de HpaA, FlaA, FlaB, uréase, UreA, UreB, hsp60, cytotoxine, cagA et VacA, et un profil SDS-PAGE distinct de celui *d'Helicobacter pylori* dans des conditions réductrices.

23. Utilisation selon la revendication 22, dans laquelle le sujet vertébré est un sujet porcin.

24. Composition selon l'une quelconque des revendications 1 à 3 ou lysat de *H. cerdo* selon la revendication 16 ou 17, pour son utilisation dans le traitement ou la prévention d'une infection à *Helicobacter* chez un sujet vertébré ; dans laquelle ledit *Helicobacter cerdo* est un isolat gastrique *d'Helicobacter* porcin qui a les caractéristiques suivantes : gram négatif, bâtons courts recourbés, schéma de croissance micro-aérophile, activité de l'enzyme uréase, activité de l'enzyme catalase, possession d'un ou plusieurs polypeptides immunogènes choisis dans le groupe constitué de HpaA, FlaA, FlaB, uréase, UreA, UreB, hsp60, cytotoxine, cagA et VacA, et un profil SDS-PAGE distinct de celui *d'Helicobacter pylori* dans des conditions réductrices.

25. Composition ou lysat de *H. cerdo* pour son utilisation selon la revendication 24, dans laquelle ladite composition ou ledit lysat de *H. cerdo* est en une quantité thérapeutiquement efficace.

26. Composition ou lysat de *H. cerdo* pour son utilisation selon la revendication 25, dans laquelle ledit sujet vertébré est un sujet porcin.

27. Composition ou lysat de *H. cerdo* pour son utilisation selon l'une quelconque des revendications 24 à 26, dans laquelle ladite composition ou ledit lysat de *H. cerdo* est administré(e) par voie parentérale.

28. Composition comprenant un lysat *d'Helicobacter cerdo* selon la revendication 16 ou 17 pour son utilisation dans un procédé de production d'une réponse immunologique contre *Helicobacter* chez un porcin.
